(19)

Europäisches
Patentamt
European
Patent Office
Office européen
des brevets

(11) **EP 4 269 592 A1**

(12) **EUROPEAN PATENT APPLICATION**
published in accordance with Art. 153(4) EPC

(43) Date of publication:
**01.11.2023 Bulletin 2023/44**

(21) Application number: **21911024.4**

(22) Date of filing: **24.12.2021**

(51) International Patent Classification (IPC):
*C12N 15/54* (2006.01)    *C12M 1/00* (2006.01)
*C12N 1/15* (2006.01)    *C12N 1/19* (2006.01)
*C12N 1/21* (2006.01)    *C12N 5/10* (2006.01)
*C12N 9/12* (2006.01)    *C12N 15/63* (2006.01)
*C12Q 1/6844* (2018.01)

(52) Cooperative Patent Classification (CPC):
**C12M 1/00; C12N 5/10; C12N 9/10; C12N 9/12;
C12N 15/63; C12N 15/74; C12N 15/80;
C12N 15/81; C12Q 1/6844**

(86) International application number:
**PCT/JP2021/048076**

(87) International publication number:
**WO 2022/138887 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.12.2020   JP 2020216600**

(71) Applicant: **Nippon Gene Co., Ltd**
**Chiyoda-ku, Tokyo 101-0054 (JP)**

(72) Inventors:
• **ASANO Shizuko**
  **Toyama-shi, Toyama 930-0834 (JP)**
• **KOKUTANI Ryota**
  **Toyama-shi, Toyama 930-0834 (JP)**
• **MINEGISHI Yasutaka**
  **Toyama-shi, Toyama 930-0834 (JP)**

• **MAKI Fuminori**
  **Toyama-shi, Toyama 930-0834 (JP)**
• **IZAWA Masaki**
  **Toyama-shi, Toyama 930-0834 (JP)**
• **YONEDA Yuko**
  **Toyama-shi, Toyama 930-0834 (JP)**

(74) Representative: **Michalski Hüttermann & Partner
Patentanwälte mbB
Kaistraße 16A
40221 Düsseldorf (DE)**

Remarks:
•A request for correction of the claims has been filed pursuant to Rule 139 EPC. A decision on the request will be taken during the proceedings before the examining division (Guidelines for Examination in the EPO, A-V, 3).
•The complete document including Reference Table(s) and the Sequence Listing(s) can be downloaded from the EPO website

(54)    **MODIFIED DNA POLYMERASE**

(57)    An object of the present invention is to provide a novel modified thermostable DNA polymerase having a reverse transcription activity. According to the present invention, there is provided a DNA polymerase having an activity to catalyze a reverse transcription reaction in the presence of $Mg^{2+}$ and comprising an amino acid sequence having an identity of 90% or higher to the amino acid sequence of SEQ ID NO: 5 and amino acid substitutions at both positions 744 and 745 with positively charged amino acids.

EP 4 269 592 A1

**(Cont. next page)**

[Figure 4-1]

WT (EA)          **Delta Rn vs. Cycle**

Cycle number

[Figure 4-2]

**Delta Rn vs. Cycle**

KK

Cycle number

2            **(Cont. next page)**

[Figure 4-3]

**Delta Rn vs. Cycle**

RR

Cycle number

[Figure 4-4]

**Standard Curve**

WT (EA)

Log C0

3

[Figure 4-5]

[Figure 4-6]

**Description**

Technical Field

Cross-reference to related application

This application claims Convention priority based on Japanese Patent Application No. 2020-216600, filed on December 25, 2020, the entire description of which is expressly incorporated herein as a disclosure.

The present invention relates to a DNA polymerase having an activity to catalyze a reverse transcription reaction in the presence of $Mg^{2+}$ and comprising an amino acid sequence having an identity of 90% or higher to the amino acid sequence of SEQ ID NO: 5 and amino acid substitutions at positions 744 and 745 with positively charged amino acids. The present invention also relates to a nucleic acid amplification method using said DNA polymerase.

Background Technique

The reverse transcription-polymerase chain reaction (RT-PCR) is a reaction in which cDNA is synthesized from RNA by a reverse transcription reaction, and then DNA fragments are amplified from the cDNA by a DNA polymerase chain reaction (PCR). RT-PCR is typically used to detect expressed genes, to generate cDNA templates for cloning and sequencing, and to detect RNA viruses (e.g., PCR tests for the diagnosis of novel coronavirus infection (COVID-19)). In RT-PCR, the reverse transcription reaction and DNA polymerase chain reaction are typically conducted in separate reaction systems, where cDNA synthesis (reverse transcription) is first performed from RNA, and then a portion of the cDNA is taken into a separate tube and amplified by PCR. In one-step RT-PCR, the cDNA synthesis from RNA (reverse transcription) and subsequent PCR proceed in the same reaction tube. One of the advantages of the one-step RT-PCR is that variation and contamination are reduced thanks to the simplified operating procedure.

As the one-step RT-PCR, those of one-enzyme type and two-enzyme type have been developed. In the case of the two-enzyme type, the reverse transcription reaction and the DNA polymerase chain reaction are carried out by separate enzymes, and for example, a mixture of a retrovirus-derived reverse transcriptase (RT) and a thermostable DNA polymerase (WO1994/008032 and Japanese Patent No. 5191041, entire disclosures of which are incorporated herein by reference) is used.

In the case of the one-enzyme type, both the reverse transcription reaction and the DNA polymerase chain reaction are carried out by one enzyme. The challenge of the one-enzyme type is selection or development of enzymes that exhibit excellent performances for both the reverse transcription reaction and the DNA polymerase chain reaction. For example, Taq polymerase, the most widely used thermostable DNA polymerase, has weak reverse transcription activity, and even in the case of modified types thereof, many of them have insufficient reverse transcription activity. Tth polymerase, a thermostable DNA polymerase like Taq polymerase, has reverse transcription activity, and has characteristics that the reverse transcription activity thereof is strong in the presence of $Mn^{2+}$, and the DNA polymerase chain reaction activity thereof is strong in the presence of $Mg^{2+}$ (T.W Myers and D.H. Gelfand, Biochem., 30:7661-7666 (1991), entire disclosure of this document is incorporated herein by reference). Myers et al., 1991 discloses RT-PCR, in which Tth DNA polymerase is used to perform the reverse transcription reaction in the presence of $Mn^{2+}$, and the DNA polymerase chain reaction in the presence of $Mg^{2+}$.

Summary of the Invention

Object to be achieved by the invention

When a thermostable DNA polymerase having a reverse transcription activity (e.g., Tth DNA polymerase) is used for one-step RT-PCR, either the reverse transcription activity or the DNA polymerase chain reaction activity is not fully obtained depending on the type of divalent cation contained in the reaction solution, and therefore, for example, a step of adding a reaction solution containing $Mg^{2+}$ for PCR after the reverse transcription reaction is necessary. To further simplify the operating procedure, there was a need for a thermostable DNA polymerase that exhibits both reverse transcription activity and DNA polymerase chain reaction activity in the presence of divalent cation of the same type or a thermostable DNA polymerase that exhibits reverse transcription activity in the presence of $Mg^{2+}$.

Therefore, an object of the present invention is to provide a novel modified thermostable DNA polymerase having a reverse transcription activity.

Means for achieving the object

The inventors of the present invention conducted various researches in order to achieve the aforementioned object, and as a result, found that a modified thermostable DNA polymerase created by introducing mutations into the amino acid sequence of the wild-type Tth DNA polymerase at positions 744 and 745 has a reverse transcription activity in the presence of $Mg^{2+}$. Furthermore, they also found that the modified thermostable DNA polymerase of the present invention favorably amplifies nucleic acids from both RNA templates and DNA templates in the nucleic acid amplification reaction in the presence of $Mg^{2+}$. The present invention was accomplished on the basis of the above findings.

The present invention provides the following inventions.

[1] A DNA polymerase having an activity to catalyze a reverse transcription reaction in the presence of $Mg^{2+}$ and comprising an amino acid sequence having an identity of 90% or higher to the amino acid sequence of SEQ ID NO: 5 and amino acid substitutions at both positions of 744 and 745 with positively charged amino acids.

[2] The DNA polymerase according to [1], wherein:

the amino acid substitution at position 744 is E744K, E744R or E744H, and
the amino acid substitution at position 745 is A745K, A745R or A745H.

[3] A DNA polymerase having an activity to catalyze a reverse transcription reaction in the presence of $Mg^{2+}$ and comprising an amino acid sequence with amino acid substitutions at both positions of 744 and 745 in the amino acid sequence of SEQ ID NO: 5, wherein:

the amino acid substitution at position 744 is E744K or E744R, and
the amino acid substitution at position 745 is A745K or A745R.

[4] The DNA polymerase according to any one of [1] to [3] having a further amino acid substitution at position 683 in the amino acid sequence the amino acid sequence of SEQ ID NO: 5.

[5] The DNA polymerase according to [4], wherein the amino acid substitution at position 683 is E683K, E683L or E683Y.

[6] A method for amplifying a nucleic acid, which comprises:

(a) preparing a reaction solution containing a nucleic acid template, the DNA polymerase according to any one of [1] to [5], and $Mg^{2+}$, and
(b) subjecting the reaction solution of (a) to a temperature cycle to amplify a DNA molecule complementary to a part or all of the nucleic acid template.

[7] The method according to [6], wherein in the step of (a), the reaction solution further contains another thermostable DNA polymerase.

[8] The method according to [6] or [7], which further comprises (c) monitoring progress of the nucleic acid amplification reaction in real time.

[9] The method according to any one of [6] to [8], wherein the nucleic acid amplification reaction is a one-step RT-PCR.

[10] A composition containing the DNA polymerase according to any one of [1] to [5].

[11] A kit comprising the DNA polymerase according to any one of [1] to [5] and $Mg^{2+}$.

[12] A nucleic acid encoding the DNA polymerase according to any one of [1] to [5],

[13] An expression vector or host cell containing the nucleic acid according to [12].

[14] A DNA polymerase having an activity to catalyze a reverse transcription reaction in the presence of $Mg^{2+}$ and comprising an amino acid sequence having an identity of 90% or higher to the amino acid sequence of SEQ ID NO: 5, amino acid substitution at position 683 with lysine, leucine or tyrosine, and amino acid substitutions at both positions of 744 and 745 with positively charged amino acids.

[15] The DNA polymerase according to [14], wherein the amino acid substitution at position 683 is E683K, E683L or E683Y, the amino acid substitution at position 744 is E744K, E744R or E744H, and the amino acid substitution at position 745 is A745K, A745R or A745H.

[16] A DNA polymerase having an activity to catalyze a reverse transcription reaction in the presence of $Mg^{2+}$, and comprising an amino acid sequence with amino acid substitutions at both positions of 683, 744 and 745 in the amino acid sequence of SEQ ID NO: 5, wherein:

the amino acid substitution at position 683 is E683K,
the amino acid substitution at position 744 is E744K or E744R; and
the amino acid substitution at position 745 is A745K or A745R.

Brief Description of Drawings

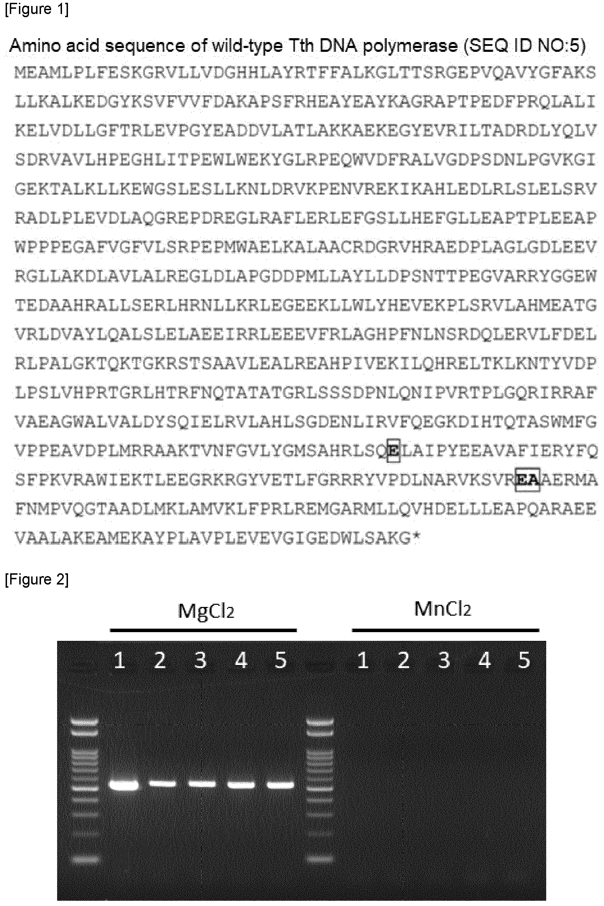

[Fig. 1] Fig. 1 shows the amino acid sequence of the wild-type Tth DNA polymerase (SEQ ID NO: 5). The amino acid residues EA at positions 744 and 745 of the amino acid sequence, in which the mutations were introduced in Example 1, are indicated in bold and with enclosing line. In addition, amino acid residue E at position 683 of the amino acid sequence, in which the mutation was introduced in Example 7, is shown in bold and with enclosing line.

[Fig. 2] Fig. 2 shows the results of PCR in which a 520 bp portion of $\lambda$DNA was amplified by using the wild-type Tth DNA polymerase (EA) and modified Tth DNA polymerases (RA, ER, RR, and KK) with $MgCl_2$ or $MnCl_2$. The numbers in the figure indicate the types of the wild-type and modified polymerases: 1, EA (wild-type); 2, RA; 3, ER; 4, RR; and 5, KK. The ladder markers were 100 bp DNA ladders.

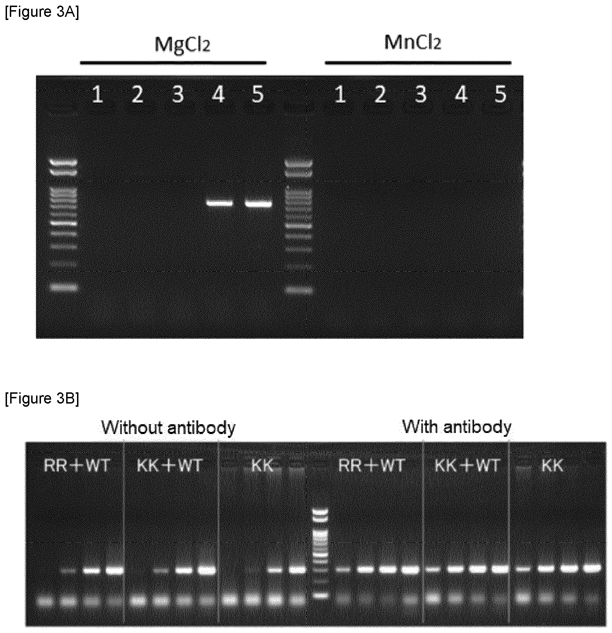

[Fig. 3A] Fig. 3A shows the results of PCR in which a 734 bp portion of $\beta$-actin was amplified by using the wild-type Tth DNA polymerase (EA) and modified Tth DNA polymerases (RA, ER, RR, and KK) with $MgCl_2$ or $MnCl_2$. The numbers in the figure indicate the types of the wild-type and modified polymerases: 1, EA (wild-type); 2, RA; 3, ER; 4, RR; and 5, KK. The ladder markers were 100 bp DNA ladders.

[Fig. 3B] Fig. 3B shows the difference in amplifications performed without adding antibody (without antibody) and with adding antibody (with antibody). The enzymes used were a combination of RR type modified DNA polymerase and the wild-type Tth DNA polymerase (RR + WT), a combination of KK type modified DNA polymerase and the wild-type Tth DNA polymerase (KK + WT), and the KK type modified Tth DNA polymerase (KK). The amounts of the template were 0.1 ng, 1 ng, 10 ng and 100 ng from the leftmost lane for each enzyme.

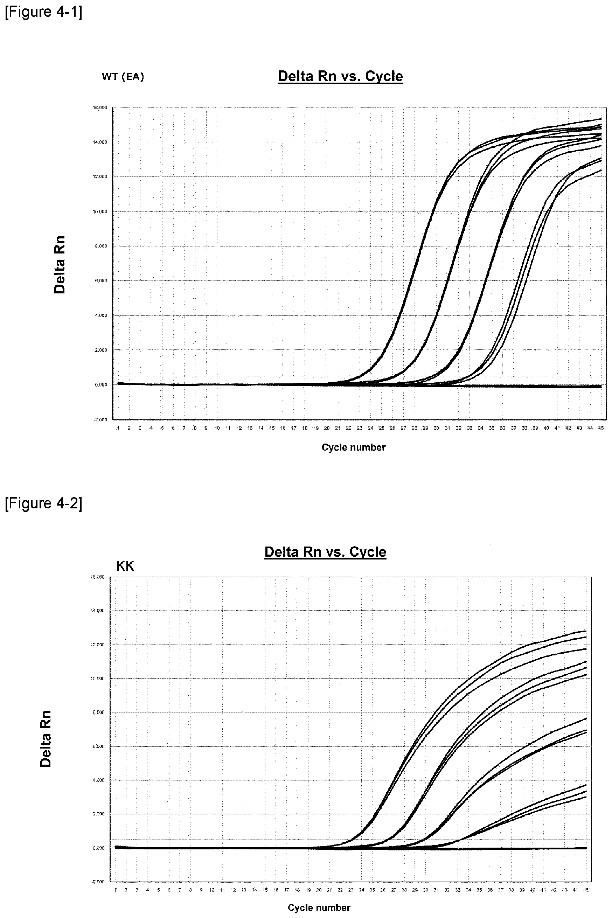

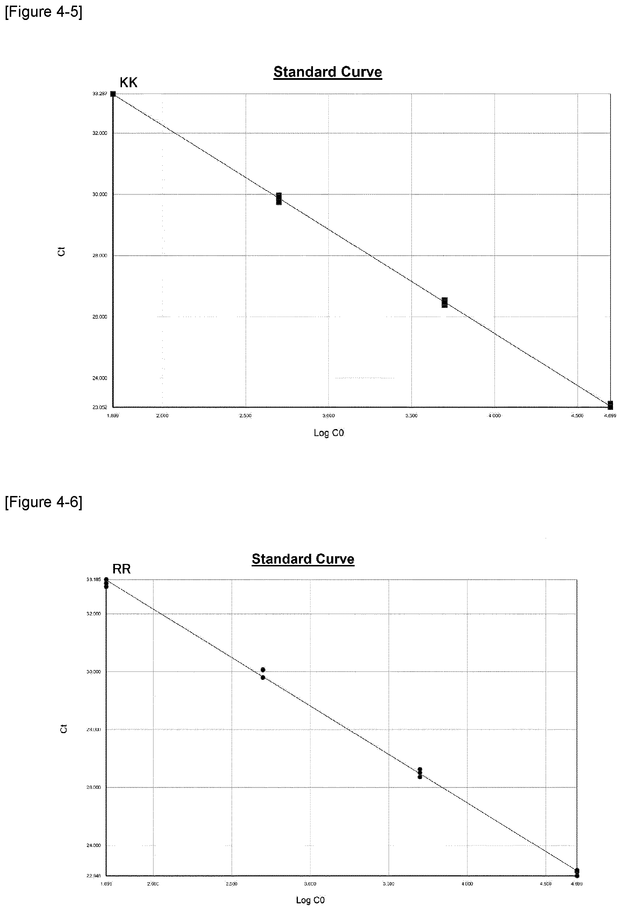

[Fig. 4-1] Figs. 4-1 to 4-6 show the amplification curves and calibration curves of the real-time PCR performed in the SARS-CoV-2 detection system in Example 4 using each Tth DNA polymerase in the presence of $MgCl_2$ and anti-Taq antibody. Fig. 4-1 shows the amplification curves of real-time PCR performed by using the wild-type Tth DNA polymerase (WT).

[Fig. 4-2] Fig. 4-2 shows the amplification curves of real-time PCR performed by using the KK type modified Tth DNA polymerase.

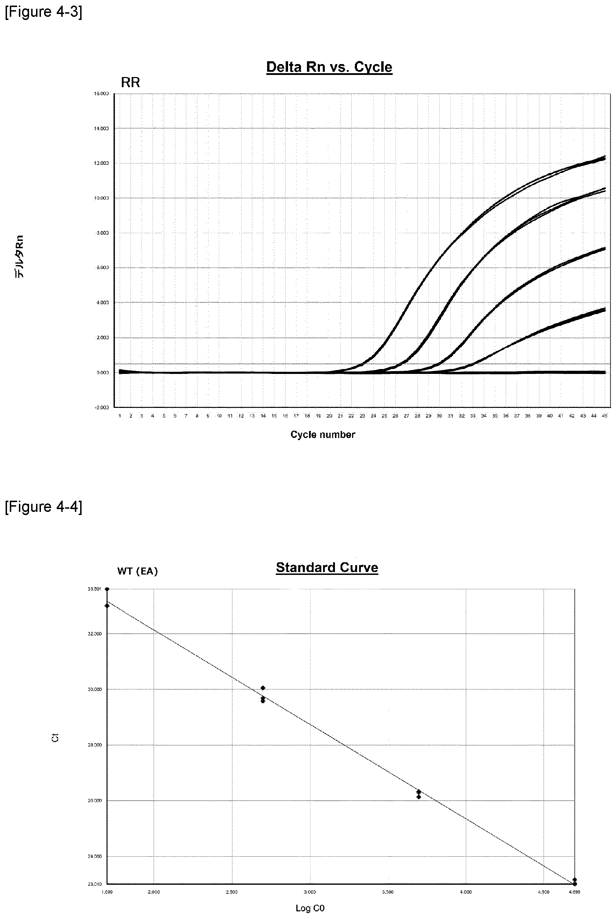

[Fig. 4-3] Fig. 4-3 shows the amplification curves of real-time PCR performed by using the RR type modified Tth DNA polymerase.

[Fig. 4-4] Fig. 4-4 shows the calibration curve for PCR data of real-time PCR performed by using the wild-type Tth DNA polymerase (WT).

[Fig. 4-5] Fig. 4-5 shows the calibration curve for PCR data of real-time PCR performed by using the KK type modified Tth DNA polymerase.

[Fig. 4-6] Fig. 4-6 shows the calibration curve for PCR data of real-time PCR performed by using the RR type modified Tth DNA polymerase.

[Fig. 5-1] Figs. 5-1 to 5-5 show the amplification curves and calibration curves of the one-step RT-qPCR performed in the SARS-CoV-2 detection system in Example 5 by using each Tth DNA polymerase in the presence of $MgCl_2$. Fig. 5-1 shows the amplification curves of the one-step RT-qPCR performed by using the wild-type Tth DNA polymerase (WT).

[Fig. 5-2] Fig. 5-2 shows the amplification curves of the one-step RT-qPCR performed by using the KK type modified Tth DNA polymerase.

[Fig. 5-3] Fig. 5-3 shows the amplification curves of the one-step RT-qPCR performed by using the RR type modified Tth DNA polymerase.

[Fig. 5-4] Fig. 5-4 shows the calibration curve for the data of the one-step RT-qPCR performed by using the KK type modified Tth DNA polymerase.

[Fig. 5-5] Fig. 5-5 shows the calibration curve for the data of the one-step RT-qPCR performed by using the RR type modified Tth DNA polymerase.

[Fig. 6-1] Figs. 6-1 to 6-3 show the amplification curves of one-step RT-qPCR performed in the SARS-CoV-2 detection system in Example 5 by using each Tth DNA polymerase in the presence of $MnCl_2$. Fig. 6-1 shows the amplification curves of the one-step RT-qPCR performed by using the wild-type Tth DNA polymerase (WT).

[Fig. 6-2] Fig. 6-2 shows the amplification curves of the one-step RT-qPCR performed by using the KK type modified Tth DNA polymerase.

[Fig. 6-3] Fig. 6-3 shows the amplification curves of one-step RT-qPCR performed by using the RR type modified Tth DNA polymerase.

[Fig. 7-1] Figs. 7-1 to 7-5 show the amplification curves of one-step RT-qPCR performed in the SARS-CoV-2 detection system in Example 6 by using each Tth DNA polymerase in the presence of $MgCl_2$ and anti-Taq antibody. Fig. 7-1 shows the amplification curves of the one-step RT-qPCR performed by using the wild-type Tth DNA polymerase (WT).

[Fig. 7-2] Fig. 7-2 shows the amplification curves of the one-step RT-qPCR performed by using the KK type modified Tth DNA polymerase.

[Fig. 7-3] Fig. 7-3 shows the amplification curves of the one-step RT-qPCR performed by using the RR type modified Tth DNA polymerase.

[Fig. 7-4] Fig. 7-4 shows the calibration curve for the one-step RT-qPCR data obtained by using the KK type modified Tth DNA polymerase.

[Fig. 7-5] Fig. 7-5 shows the calibration curve for the one-step RT-qPCR data obtained by using the RR type modified Tth DNA polymerase.

[Fig. 8-1] Figs. 8-1 to 8-3 show the amplification curves of one-step RT-qPCR performed in the SARS-CoV-2 detection system in Example 6 by using each Tth DNA polymerase in the presence of $MnCl_2$ and anti-Taq antibody. Fig. 8-1 shows the amplification curves of one-step RT-qPCR performed by using the wild-type Tth DNA polymerase (WT).

[Fig. 8-2] Fig. 8-2 shows the amplification curves of the one-step RT-qPCR performed by using the KK type modified Tth DNA polymerase.

[Fig. 8-3] Fig. 8-3 shows the amplification curves of one-step RT-qPCR performed by using the RR type modified Tth DNA polymerase.

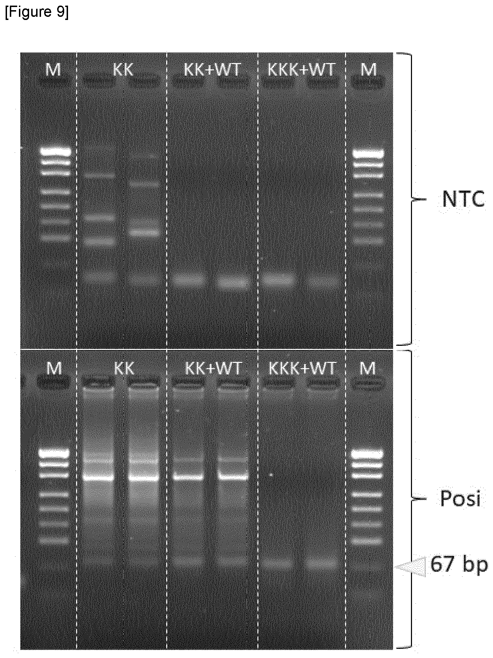

[Fig. 9] Fig. 9 shows the results of RT-PCR performed in the SARS-CoV-2 detection system in the presence of $MgCl_2$. The amplified strand length of the template was 67 bp. The DNA polymerases used were as follows: KK, KK type modified Tth DNA polymerase; KK+WT, combination of KK type modified Tth DNA polymerase and wild-type Tth DNA polymerase; and KKK+WT, combination of the KKK type modified Tth DNA polymerase and wild-type Tth DNA polymerase. In the figure, NTC stands for the results for the negative control, Posi stands for the results for purified RNA extracted from spiked samples as template, and M stands for pUC19/MspI digest.

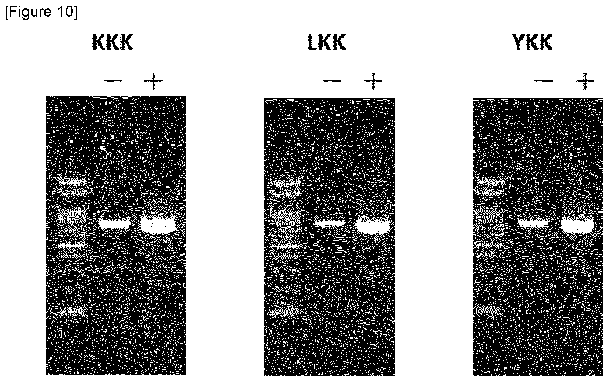

[Fig. 10] Fig. 10 shows the results of RT-PCR in which a 734 bp portion of β-actin was amplified in the presence of MgClz. KKK; KKK type modified Tth DNA polymerase alone (-), and combination of KKK type modified Tth DNA polymerase and the wild-type Tth DNA polymerase (+): LKK; LKK type modified Tth DNA polymerase alone (-), and combination of LKK type modified Tth DNA polymerase and the wild-type Tth DNA polymerase (+): and YKK; YKK type modified Tth DNA polymerase alone (-), and combination of YKK type modified Tth DNA polymerase and the wild-type Tth DNA polymerase (+). The ladder markers were 100 bp DNA ladders.

Modes for Carrying out the Invention

The explanations of the present invention described below may be based on representative embodiments or specific examples, but the present invention is not limited to such embodiments. In this description, numerical value ranges expressed with "to" mean a range that includes the numerical values mentioned before and after "to" as the minimum and maximum values.

<Modified DNA polymerase>

(Amino acid substitutions at positions 744 and 745)

The DNA polymerase of the present invention has an activity to catalyze a reverse transcription reaction in the presence of $Mg^{2+}$ (reverse transcription activity) and contains an amino acid sequence that shows an identity of 90% or higher to the amino acid sequence of SEQ ID NO: 5 in which the amino acids at both positions 744 and 745 are replaced with positively charged amino acids.

The present invention relates to a thermostable DNA polymerase having a reverse transcription activity. Examples of such a thermostable DNA polymerase having a reverse transcription activity include a DNA polymerase derived from *Thermus thermophilus* HB8, DNA polymerase derived from *Thermus thermophilus* HB27, DNA polymerase derived from *Thermus thermophilus* OM72 described in the examples mentioned below, DNA polymerase derived from *Thermus* sp. Z05 (Z05), and so forth, but are not limited to these. The amino acid sequences of thermostable DNA polymerases having a reverse transcription activity of *T. thermophilus* are provided from GenBank under the following accession numbers: HB8 strain, BAA06033.1; and HB27 strain, AAS81038.1. The nucleotide sequences encoding these amino acid sequences are provided by GenBank under the following accession numbers: HB8 strain, D28878.1; and HB27 strain, AE017221.1 (671122-673626). The thermostable DNA polymerase having a reverse transcription activity is preferably a thermostable DNA polymerase derived from *Thermus thermophilus* (referred to as Tth DNA polymerase in this description). The reverse transcription activity of the wild-type Tth DNA polymerase is strong in the presence of $Mn^{2+}$, and the DNA polymerase chain reaction activity of the same is strong in the presence of $Mg^{2+}$. The Tth DNA polymerase lacks 3' to 5' exonucleolytic proofreading activity.

As described above, the reverse transcription activity of the wild-type Tth DNA polymerase is strong in the presence of manganese ions, and the DNApolymerase chain reaction activity of the same is strong in the presence of magnesium ions. As shown in Example 3 mentioned below, in RT-PCR conducted by using the wild-type Tth DNA polymerase, no bands of nucleic acid amplification reaction products could be confirmed with either the reaction solution containing magnesium ions or the reaction solution containing manganese ions. This is considered to be because RNA-dependent reverse transcription reaction hardly occurred in the reaction solution containing magnesium ions. That is also considered to be because even if cDNA was synthesized by the reverse transcription reaction in the reaction solution containing magnesium ions, DNA was hardly amplified from the cDNA and thus was not amplified to such a level that it was observable as a band. On the other hand, the inventors of the present invention surprisingly found that by RT-PCR performed by using a modified DNA polymerase having an amino acid sequence derived form the amino acid sequence of SEQ ID NO: 5 by amino acid replacements of positively charged amino acids for the amino acids at both positions 744 and 745 in a reaction solution containing magnesium ions, an amplification product that could be confirmed as a band can be produced, i.e., DNA can be amplified from an RNA template. This is considered to be because the modified Tth DNA polymerase mentioned above has both reverse transcription activity and DNA polymerase activity in the reaction solution containing magnesium ions.

The description "having an activity to catalyze reverse transcription reaction in the presence of $Mg^{2+}$" used in this description means that the DNA polymerase catalyzes the reverse transcription reaction so that cDNA is synthesized from the RNA template in the reaction solution containing magnesium ions. The aforementioned reaction solution is substantially free of manganese ions, and it is supposed that the reverse transcription reaction is dependent on the action of magnesium ions. The activity of DNA polymerase to catalyze the reverse transcription reaction in the presence of $Mg^{2+}$ can be verified by, for example, performing an isothermal reaction at 65°C for 10 minutes in a composition of 20mM Tris-HCl (pH 8.8), 50mM KCl, 0.1% Tween 20, 0.1 mg/mL acetylated BSA, 0.5% DMSO, 1 mM DTT, 500 mM trehalose, and 2 mM $MgCl_2$ using tritiated thymidine, polyadenylate as the template, and pentadecathymidylate as the primer on a heat block or the like, and detecting the synthesis of polythymidylate using the liquid scintillation system LSC-6101 (Aloka). Alternatively, it can also be verified by performing RT-PCR under the conditions of Example 3 using total RNA extracted and purified from mouse kidney as the template and primers for mouse β-actin amplification (SEQ ID NOS: 16 and 17) in the presence of 2 mM $MgCl_2$ on a thermal cycler, subjecting a part of the reaction solution to gel electrophoresis after completion of the reaction, performing ethidium bromide staining, and confirming presence or absence of amplification of the target DNA fragment under UV irradiation. The DNA polymerase of the present invention has an activity to catalyze a reverse transcription reaction in the presence of $Mg^{2+}$. The DNA polymerase of the present invention may have the DNA polymerase activity in the presence of $Mg^{2+}$ at the same level as the wild type or a reduced level compared with the wild type. Alternatively, it may lack the DNA polymerase activity at least in the presence of $Mg^{2+}$.

The DNA polymerase of the present invention is a modified DNA polymerase having (i) the amino acid sequence of SEQ ID NO: 5 or (ii) amino acid sequence having an amino acid identity of 90% or higher to the amino acid sequence of SEQ ID NO: 5, in which both amino acids at positions 744 and 745 are replaced with positively charged amino acids. When the positions of the amino acid residues are displaced by deletion or insertion of amino acids in (ii) the amino acid sequence having an amino acid identity of 90% or higher to the amino acid sequence of SEQ ID NO: 5, the DNA polymerase of the present invention is such a modified DNA polymerase in which both of the two amino acid residues at the positions corresponding to positions 744 and 745 of the amino acid sequence of SEQ ID NO: 5 are replaced with positively charged amino acids. The amino acid sequence of SEQ ID NO: 5 described in this description is identical to that of the DNA polymerase of the strain HB27, GenBank accession number AAS81038.1. The amino acid sequence having an amino acid identity of 90% or higher to the amino acid sequence of SEQ ID NO: 5 may be, for example, the thermostable DNA polymerase derived from *Thermus thermophilus* HB8 or the DNA polymerase derived from *Thermus* sp. Z05 (Z05).

The amino acids at positions 744 and 745 of the protein consisting of the amino acid sequence of SEQ ID NO: 5 are the 744th and 745th amino acids, respectively, counted from the N-terminus of the amino acid sequence of SEQ ID NO: 5. In the amino acid sequence of SEQ ID NO: 5, the unmodified amino acids at positions 744 and 745 (wild type) are glutamic acid (E, Glu) and alanine (A, Ala), respectively. The unmodified amino acids can be described as E744 and A745 by indicating the codes of the amino acids on the left (head) of the numbers indicating the positions of the amino acids. The positively charged amino acid may be, for example, arginine (R, Arg), lysine (K, Lys), or histidine (H, His).

In one embodiment of the present invention, the modified DNA polymerase has an amino acid substitution of E744K, E744R or E744H at position 744, and an amino acid substitution of A745K, A745R or A745H at position 745. The references of E744K, E744R and E744H mean that the 744th glutamic acid of the amino acid sequence of SEQ ID NO: 5 counted from the N-terminus thereof is replaced with lysine, arginine, or histidine, respectively. Similarly, the references of A745K, A745R, and A745H mean that the alanine at position 745 of the amino acid sequence of SEQ ID NO: 5 counted from the N-terminus thereof is replaced with lysine, arginine, or histidine, respectively. In these references, the amino acids present before the modification are indicated on the left side (head) of the numbers indicating the positions of the amino acids, and the amino acids present after the modification on the right side (back) of the numbers indicating the positions of the amino acids. The amino acid substitutions at positions 744 and 745 of the amino acid sequence of SEQ ID NO: 5 can be selected from E744K and A745K, E744K and A745R, E744K and A745H, E744R and A745K, E744R and A745R, E744R and A745H, E744H and A745K, E744H and A745R, and E744H and A745H.

The amino acid substitutions at positions 744 and 745 of the amino acid sequence of SEQ ID NO: 5 are preferably chosen to be either one of E744K and A745K, E744K and A745R, E744R and A745K, and E744R and A745R, more preferably either E744K and A745K or E744R and A745R.

So long as the amino acid sequence of the DNA polymerase of the present invention has an activity to catalyze a reverse transcription reaction in the presence of $Mg^{2+}$, it may have mutations compared with the amino acid sequence of SEQ ID NO: 5 in addition to the amino acid substitutions at positions 744 and 745 of the amino acid sequence of SEQ ID NO: 5, and such mutations may be an artificially introduced modification or may be a mutation occurring due to that the wild-type DNA polymerase from which the modified DNA polymerase is derived is derived from a different species or strain other than *Thermus thermophilus* HB27. The amino acid sequence of the DNA polymerase of the present invention may have any level of amino acid sequence identity to the amino acid sequence of SEQ ID NO: 5 so long as it has an activity to catalyze the reverse transcription reaction in the presence of $Mg^{2+}$, but it preferably comprises or consists of an amino acid sequence having an amino acid sequence identity of 91% or higher, 92% or higher, 93% or higher, 94% or higher, 95% or higher, 96% or higher, 97% or higher, 98% or higher, or 99% or higher to the amino acid sequence of SEQ ID NO: 5. The amino acid sequence identity values can be calculated for the sequence including the substitutions at positions 744 and 745. The value of the amino acid sequence identity of the amino acid sequence including the substitutions is defined as the percentage of amino acid residues that are identical between two aligned amino acid sequences to be compared, wherein gaps may be introduced as required in order to obtain the maximum sequence identity. The amino acid sequence identity can be determined by using publicly available computer software such as, for example, BLAST, BLAST-2, ALIGN, and Megalign (DNASTAR) software.

Mutations or modifications that can be additionally introduced into the DNA polymerase of the present invention include, for example, but not limited to, a mutation that deletes an N-terminal region of DNA polymerase derived from *Thermus thermophilus* to inactivate the 5'→3' exonuclease activity (Arakawa et al., DNA Research 3, 87-92, 1996, the entire description of this reference is incorporated into this description by reference), modification that links a single-strand DNA binding protein, e.g., RB69SSB, to the DNA polymerase with a linker (Sun et al., Proteins 2006 Oct 1;65(1):231-8, doi: 10.1002/prot.21088, the entire description of this reference is incorporated into this description by reference), modification that links a doublestranded DNA binding protein, e.g., Sso7d, to the DNA polymerase to prevent the polymerase from slipping off from the DNA strand and to improve ability for continuous synthesis thereof (Wang et al., Nucleic Acids Research, 2004, Vol. 32, No. 3 1197-1207, DOI: 10.1093/nar/gkh271, the entire description of this reference is incorporated into this description by reference), and so forth.

In a particular embodiment, the DNA polymerase of the present invention is a DNA polymerase having an activity to catalyze a reverse transcription reaction in the presence of $Mg^{2+}$, and comprising an amino acid sequence with amino acid substitutions at both positions of 744 and 745 in the amino acid sequence of SEQ ID NO: 5, wherein the amino acid substitution at position 744 is E744K or E744R, and the amino acid substitution at position 745 is A745K or A745R. In a particular embodiment, the DNA polymerase of the present invention is a DNA polymerase having an activity to catalyze a reverse transcription reaction in the presence of $Mg^{2+}$, and comprising an amino acid sequence with amino acid substitutions of E744K and A745K in the amino acid sequence of SEQ ID NO:5. In a particular embodiment, the DNA polymerase of the present invention is a DNA polymerase having an activity to catalyze a reverse transcription reaction in the presence of $Mg^{2+}$, and comprising an amino acid sequence with amino acid substitutions of E744K and A745R in the amino acid sequence of SEQ ID NO:5. In a particular embodiment, the DNA polymerase of the present invention is a DNA polymerase having an activity to catalyze a reverse transcription reaction in the presence of $Mg^{2+}$, and comprising an amino acid sequence with amino acid substitutions of E744R and A745K in the amino acid sequence of SEQ ID NO:5. In these embodiments, the amino acid residues at positions other than positions 744 and 745 may be the native amino acid residues of the amino acid sequence of SEQ ID NO: 5.

(Amino acid substitution at position 683)

In another embodiment, the DNA polymerase of the present invention may contain a further amino acid substitution at position 683 of the amino acid sequence of SEQ ID NO: 5. The amino acid residue at position 683 of the protein consisting of the amino acid sequence of SEQ ID NO: 5 is the 683rd amino acid counted from the N-terminus of the amino acid sequence of SEQ ID NO: 5. In the amino acid sequence of SEQ ID NO: 5, the 683rd amino acid present before the modification (wild type) is glutamic acid (E, Glu). The amino acid residue at position 683 can be replaced with an amino acid residue that does not affect the reverse transcription reaction catalyzed by the DNA polymerase having an activity to catalyze a reverse transcription reaction in the presence of $Mg^{2+}$. This substitution may also reduce the DNA polymerase chain reaction activity of the DNA polymerase having an activity to catalyze a reverse transcription activity in the presence of $Mg^{2+}$, and containing an amino acid sequence having amino acid substitutions at both positions 744 and 745. Glutamic acid at position 683 may be replaced with, for example, but not limited to, lysine (K, Lys), leucine (L, Leu), or tyrosine (Y, Tyr).

The inventors of the present invention found that such a further amino acid substitution at position 683 of the amino acid sequence of SEQ ID NO: 5 reduces the DNA polymerase chain reaction activity of the DNA polymerase of the present invention having an activity to catalyze a reverse transcription reaction in the presence of $Mg^{2+}$, and containing an amino acid sequence having amino acid substitutions at both positions 744 and 745. The DNA polymerase of the present invention having a further amino acid substitution at position 683 of the amino acid sequence of SEQ ID NO: 5 can be used in combination with another thermostable DNA polymerase. As the other thermostable DNA polymerase, one having no or low reverse transcription activity in the presence of $Mg^{2+}$ can be selected. The other thermostable DNA polymerase can be selected from, for example, Taq DNA polymerase, Pfu DNA polymerase, Tth DNA polymerase having no reverse transcription activity in the presence of $Mg^{2+}$ (e.g., the wild-type Tth DNA polymerase, recombinant Tth DNA polymerase having no reverse transcription activity in the presence of $Mg^{2+}$, etc.), and combinations of these. Such combinations with another thermostable DNA polymerase enable more accurate detection of the target.

The DNA polymerase of the present invention may contain an amino acid sequence having an activity to catalyze a reverse transcription reaction in the presence of $Mg^{2+}$ (reverse transcription activity), and having an identity of 90% or higher to the amino acid sequence of SEQ ID NO: 5, wherein the amino acid at position 683 is replaced with lysine, leucine or tyrosine, and amino acids at both positions 744 and 745 are replaced with positively charged amino acids. The amino acid sequence of the DNA polymerase of the present invention may have any level of amino acid sequence identity to the amino acid sequence of SEQ ID NO: 5 so long as it has an activity to catalyze a reverse transcription reaction in the presence of $Mg^{2+}$, but it preferably contains or consists of an amino acid sequence having an amino acid sequence identity of 91% or higher, 92% or higher, 93% or higher, 94% or higher, 95% or higher, 96% or higher, 97% or higher, 98% or higher, or 99% or higher to the amino acid sequence of SEQ ID NO: 5. The value of amino acid sequence identity can be calculated for the sequence including the substitutions at positions 683, 744 and 745.

In one embodiment of the present invention, the modified DNA polymerase has the amino acid substitution of E683K, E683L or E683Y at position 683. The descriptions E683K, E683L and E683Y indicate that the glutamic acid at position 683 counted from the N-terminal of the amino acid sequence of SEQ ID NO: 5 is replaced with lysine, leucine or tyrosine, respectively. In these references, the amino acids present before the modification are indicated on the left side (head) of the number indicating the position of the amino acid, and the amino acids present after the modification on the right side (back) of the number indicating the position of the amino acid. The combination of the amino acid substitution at position 683 and the amino acid substitutions at positions of 744 and 745 of the amino acid sequence of SEQ ID NO: 5 can be chosen from, for example, E683K/E744K/A745K, E683K/E744K/A745R, E683K/E744K/A745H, E683K/E744R/A745K, E683K/E744R/A745R, E683K/E744R/A745H, E683K/E744H/A745K, E683K/E744H/A745R, E683K/E744H/A745H, E683L/E744K/A745K, E683L/E744K/A745R, E683L/E744K/A745H, E683L/E744R/A745K, E683L/E744R/A745R, E683L/E744R/A745H, E683L/E744H/A745K, E683L/E744H/A745R, E683L/E744H/A745H, E683Y/E744K/A745K, E683Y/E744K/A745R, E683Y/E744K/A745H, E683Y/E744R/A745K, E683Y/E744R/A745R, E683Y/E744R/A745H, E683Y/E744H/A745K, E683Y/E744H/A745R, and E683Y/E744H/A745H. For example, in "E683K/E744K/A745K", "/" means "and", and this description specifically means "E683K and E744K and A745K". The amino acid substitutions at positions 683, 744 and 745 of the amino acid sequence of SEQ ID NO: 5 are preferably chosen from E683K/E744K/A745K, E683K/E744K/A745R, E683K/E744R/A745K, and E683K/E744R/A745R, more preferably chosen from E683K/E744K/A745K and E683K/E744R/A745R.

In a particular embodiment, the DNA polymerase of the present invention is a DNA polymerase having an activity to catalyze a reverse transcription reaction in the presence of $Mg^{2+}$, and comprising an amino acid sequence with amino acid substitutions at positions 683, 744 and 745 in the amino acid sequence of SEQ ID NO:5, wherein the amino acid substitution at position 683 is E683K, the amino acid substitution at position 744 is E744K or E744R, and the amino acid substitution at position 745 is A745K or A745R. In another particular embodiment, the DNA polymerase of the present invention is a DNA polymerase having an activity to catalyze a reverse transcription reaction in the presence of $Mg^{2+}$, and comprising an amino acid sequence with amino acid substitutions of E683K, E744K and A745K in the amino acid sequence of SEQ ID NO:5. In another particular embodiment, the DNA polymerase of the present invention is a DNA polymerase having an activity to catalyze a reverse transcription reaction in the presence of $Mg^{2+}$, and comprising an amino acid sequence with amino acid substitutions of E683K, E744K and A745R in the amino acid sequence of SEQ ID NO:5. In another particular embodiment, the DNA polymerase of the present invention is a DNA polymerase having an activity to catalyze a reverse transcription reaction in the presence of $Mg^{2+}$, and comprising an amino acid sequence with amino acid substitutions of E683K, E744R and A745K in the amino acid sequence of SEQ ID NO:5. In these embodiments, the amino acid residues at positions other than positions 683, 744 and 745 may be native amino acid residues of the amino acid sequence of SEQ ID NO: 5.

The efficiency of the nucleic acid amplification reaction using the modified DNA polymerase of the present invention can be evaluated by performing a dilution series experiment using standard samples. For example, the amplification efficiency value (PCR efficiency) can be obtained from slope of a calibration curve. The PCR efficiency can be obtained from slope of a calibration curve, which has been drawn by plotting the results on an x-y plane, where the x-axis indicates the serially diluted nucleic acid amounts and the y-axis indicates Ct values corresponding to the nucleic acid amounts, in accordance with the following equation 1.

(Equation 1)

$$PCR\ efficiency\ (\%) = (10^{[-1/Slope]} - 1) \times 100$$

The appropriate range of the PCR efficiency is considered to be 80 to 120%, or 90 to 100% for a favorable reaction. The linearity of the calibration curve is evaluated on the basis of the correlation coefficient ($R^2$ value), of which value is preferably 0.98 or higher.

The present invention provides a nucleic acid encoding the modified DNA polymerase mentioned above or a functional fragment thereof. A functional fragment of the modified DNA polymerase of the present invention is a fragment having an activity to catalyze a reverse transcription reaction in the presence of $Mg^{2+}$. The present invention further provides an expression vector or host cell containing a nucleic acid encoding the modified DNA polymerase or a functional fragment thereof.

A DNA encoding the modified DNA polymerase of the present invention can be obtained by without any limitation, artificially introducing mutations into a DNA encoding a thermally stable DNA polymerase of the genus *Thermus* (e.g., the sequence of SEQ ID NO: 4) using any known method such as site-directed mutagenesis. A DNA encoding a thermostable DNA polymerase of the genus *Thermus* can be obtained by for example, amplifying the Tth DNA polymerase gene by PCR performed by using genomic DNA extracted from the strain HB8 or HB27 of *Thermus thermophilus* or a strain isolated from the nature and identified as belonging to the genus *Thermus* as the template, and primers synthesized on the basis of a known Tth DNA polymerase nucleotide sequence. In another method, a DNA encoding a thermostable DNA polymerase of the genus *Thermus* can be obtained by preparing a cDNA library using mRNA extracted from the strain HB8 or HB27 of *Thermus thermophilus* or a strain isolated from the nature and identified as belonging to the genus *Thermus*, and screening the cDNA library for the target DNA using a probe synthesized on the basis of a known Tth DNA polymerase nucleotide sequence.

The modified DNA polymerase of the present invention and a fragment thereof can be produced by expressing a DNA encoding the modified DNA polymerase or a fragment thereof using known methods. Expression vectors can be appropriately selected for the host cell used. Into these expression vectors, a nucleic acid encoding the modified DNA polymerase of the present invention or a fragment thereof can be inserted by known methods (methods using restriction enzymes, etc.). The expression vector of the present invention can further contain a promoter, which controls expression of the polymerase gene, replication origin, selection marker gene, and so forth. The promoter and replication origin can be appropriately chosen depending on the types of the host cell and vector. As the expression vector, plasmid vectors, phage vectors, viral vectors, etc. can be used. As the plasmid vectors, for example, plasmids derived from *E. coli* (such as pRSET, pBR322 (such as pET15b used in the examples), pBR325, pUC118, pUC119, pUC18, and pUC19), plasmids derived from *Bacillus subtilis* (e.g., pUB 110 and pTP5), and plasmids derived from yeast (e.g., YEp13, YEp24, and YCp50) can be used. As the host cell, prokaryotic cells such as those of *Escherichia coli* (*E. coli*), and *Bacillus subtilis*, and eukaryotic cells such as mammalian cells, insect cells, plant cells, and fungal cells (*Saccharomyces* sp., *Aspergillus* sp., etc.) can be used. As host organism, for example, animal individuals, plant individuals, and silkworms can be used.

The DNA polymerase can be produced by culturing host cells or host organisms under conditions suitable for each type of the cells or organisms by known methods. When *E. coli* is used as the host cell, *E. coli* can be transformed by using a DNA polymerase expression plasmid, and inoculated on an agar medium, the resulting colonies can be cultured at 37°C in a liquid medium consisting of LB medium supplemented with ampicillin and chloramphenicol until the absorbance at 600 nm reaches about 0.5, and after adding IPTG to a final concentration of 1 mM, further cultured for 4 hours to produce a Tth DNA polymerase. The bacterial cells can be suspended in 50 mM Tris-HCl (pH 8.0), 0.5 M NaCl, 1% Triton X-100, and 20 mM imidazole, enzymatically or physically (e.g., by sonication) disrupted, and centrifuged, then the supernatant can be heat-treated at 80°C, centrifuged and subjected to purification by using HisTrap HP column (Cytiva 17524802), HiTrap Q column (Cytiva 17115401), and HiTrap Heparin column (Cytiva 17040701), and finally the medium can be replaced with a storage buffer (10 mM Tris -HCl (pH 7.5), 300 mM KCl, 0.1 mM EDTA, 1 mM DTT, 0.095% Triton X-100, 50% glycerol) to obtain a Tth DNA polymerase.

<Nucleic acid amplification method>

The nucleic acid amplification method of the present invention comprises the following steps of

(a) preparing a reaction solution containing a nucleic acid template, the above modified DNA polymerase and $Mg^{2+}$, and

(b) subjecting the reaction solution of (a) mentioned above to a temperature cycle to amplify DNA molecules complementary to a part or all of the nucleic acid template.

The DNA polymerase used in the nucleic acid amplification method of the present invention is a DNA polymerase having an activity to catalyze a reverse transcription reaction in the presence of $Mg^{2+}$ (reverse transcription activity), and containing an amino acid sequence having an identity of 90% or higher to the amino acid sequence of SEQ ID NO: 5, in which amino acids at both positions 744 and 745 are replaced with positively charged amino acids, and such a DNA polymerase as described in the section of <Modified DNA polymerase> mentioned above can be used.

The modified DNA polymerase of the present invention can synthesize cDNA from an RNA template by the RNA-dependent reverse transcription activity in the presence of $Mg^{2+}$ and then amplify the DNA by the DNA-dependent DNA polymerase activity, and therefore the nucleic acid amplification method of the present invention makes it possible to carry out the reverse transcription reaction and DNA polymerase chain reaction in a single reaction system (i.e., in the same reaction solution), when RNA is used as the template. The nucleic acid amplification method of the present invention is a PCR or RT-PCR method, preferably a one-step RT-PCR method. The nucleic acid amplification method of the present invention can be used to obtain qualitative or quantitative results. The nucleic acid template is a DNA template, an RNA template, or a mixture thereof.

It is sufficient that such condition that the nucleic acid template and the DNA polymerase of the present invention can contact with each other in the presence of $Mg^{2+}$ is created by "(a) preparing a reaction solution containing a nucleic acid template, the modified DNA polymerase described above, and $Mg^{2+}$", and such a process can be attained by, but not limited to, adding the nucleic acid template and the DNA polymerase to a reaction solution containing $Mg^{2+}$-contained in a reaction tube. The reaction solution can contain reagents necessary for the nucleic acid amplification reaction, examples of such reagents include, in addition to the nucleic acid template and the DNA polymerase of the present invention, dNTPs, primers, probe, divalent cations, monovalent ions and buffer, and specifically, the divalent cations are magnesium ions, monovalent ions are ammonium ions, potassium ions, or a mixture thereof. The reaction solution can further contain a sugar such as trehalose, BSA, nonionic surfactant such as Tween 20 (registered trademark) or Brij35, and anti-DNA polymerase antibody (such as anti-Taq or anti-Tth antibody) as needed. The addition of the anti-DNA polymerase antibody to the reaction solution is considered to provide an effect of improving specificity and sensitivity (Japanese Patent Unexamined Publication (Kokai) No. 2008-17787, the entire description of which is incorporated into this description by reference).

The reaction solution is preferably substantially free of manganese ions. Although it is not intended to be bound by any specific theory, this is because manganese ions may compete with magnesium ions and inhibit the reverse transcription activity. Substantially free of manganese ions means that either no manganese ions are contained at all or very small amounts of manganese ions may be contained.

The above reaction solution can contain magnesium acetate, magnesium chloride, magnesium sulfate, magnesium carbonate, etc. as a magnesium ion source. The magnesium ion concentration in the reaction solution is in the range of 0.1 to 100 mM or 0.5 to 50 mM, preferably in the range of 1 to 10 mM, more preferably in the range of 1 to 8 mM, even more preferably in the range of 2 to 6 mM. If the magnesium ion concentration is in any of the above ranges, the reverse transcription reaction and DNA polymerase chain reaction are expected to occur, and especially in the range of 2 to 6 mM, the reverse transcription reaction and DNA polymerase chain reaction favorably occur.

When RT-PCR is performed, the primers are a combination of a primer having an antisense sequence designed to amplify a part or all of the RNA template (antisense primer) and a sequence-specific sense primer. The antisense primer is a primer for cDNA synthesis by the reverse transcription reaction, and a primer specific to the template sequence, an oligo dT primer that binds to the polyA tail of mRNA, a random primer such as a random hexamer primer, or the like can be used. In the DNA polymerase chain reaction fallowing the cDNA synthesis, a sequence-specific sense primer serves as the forward primer, and as the reverse primer, either the antisense primer used in the reverse transcription reaction or another antisense primer that can hybridize to a sequence locating upstream from the antisense primer-binding site is used. When PCR is performed, the primers may be, for example, but not limited to, sequence-specific sense or antisense primer designed to amplify a part or all of the nucleic acid template.

"(b) Subjecting the reaction solution of (a) mentioned above to a temperature cycle to amplify DNA molecules complementary to a part or all of the nucleic acid template" means, for example, subjecting the reaction solution containing the nucleic acid template, modified DNA polymerase and reagents necessary for RT-PCR to a temperature cycle suitable for the reverse transcription reaction and DNA polymerase chain reaction to amplify DNA molecules complementary to a part or all of the nucleic acid template with the primers. The conditions of the temperature cycle suitable for the nucleic acid amplification vary depending on the type of nucleic acid used as the template, characteristics of the nucleotide sequence, length to be amplified, primer sequences, and so forth, and those skilled in the art can appropriately set the conditions. When the nucleic acid amplification method is the RT-PCR method, for example, the temperature cycle can include (1) incubation for the reverse transcription reaction and (2) temperature cycling for PCR. (1) The incubation for the reverse transcription reaction can be performed at such a temperature for such a time that at least the initial cDNA synthesis can occur, and for example, incubation can be performed at any temperature in the range of 1 to 100°C for 1 second or longer. More specifically, it can be performed at any temperature in the range of 10 to 90°C, 20 to 90°C, 30 to 90°C, 40 to 90°C, 45 to 85°C, 50 to 80°C, 55 to 75°C, or 60 to 70°C for any time in the range of 1 second to 120 minutes, or 1 second to 60 minutes. It is considered that the incubation for the reverse transcription reaction is not necessarily required. The inventors of the present invention have experimentally confirmed that amplification is observed in one-step RT real-time PCR even when the reverse transcription time is 0 second (not set). This is considered to be because the reverse transcription reaction occurs in the step for the second temperature during the temperature cycle for PCR described below. As (2) the temperature cycle for PCR, for example, a temperature change (round) between two levels of a denaturation temperature of 90°C to 97°C and a second temperature of 50°C to 70°C can be performed 20 to 50 times (cycles). The temperature cycle for PCR can be performed as a three-level temperature change additionally including an extension temperature (60 to 75°C) as the case may be. When PCR is performed with a three-level temperature change, the extension temperature is generally set to be higher than the second temperature. Those skilled in the art can appropriately set the retention time for each round. For example, the retention time for each round can be set to any time of 1 second to 5 minutes, 1 second to 3 minutes, or 1 to 60 seconds. When the nucleic acid amplification method is the PCR method, (2) the temperature cycle for PCR described above can be performed, and if necessary, pre-incubation for denaturation can be performed, for example, at 90 to 97°C for 10 seconds to 15 minutes prior to the temperature cycle for PCR, as required.

In one of the embodiments of the present invention, in the RT-PCR method, the modified DNA polymerase mentioned above can be used in combination with another thermostable DNA polymerase. As the other thermostable DNA polymerase, an enzyme that has no or low reverse transcription activity in the presence of $Mg^{2+}$ can be selected. The other thermostable DNA polymerase can be selected from, for example, Taq DNA polymerase, Pfu DNA polymerase, Tth DNA polymerase not showing reverse transcription activity in the presence of $Mg^{2+}$ (e.g., the wild-type Tth DNA polymerase or recombinant Tth DNA not showing reverse transcription activity in the presence of $Mg^{2+}$) and a combination thereof. Pfu DNA polymerase has no 5'→3' exo activity, and therefore it cannot be used as a single enzyme in the probing method, but it is said that, in general PCR methods, a combination of a family A type such as Taq DNA polymerase with a family B type such as Pfu DNA polymerase enables amplification of long-strand DNA and improves sensitivity, and the same effect can be expected also in one-step PCR performed by using RNA as the template.

That is, the present invention provides a method for amplifying a nucleic acid, which comprises the following steps (a) and (b):

(a) preparing a reaction solution containing a nucleic acid template, the DNA polymerase, another thermostable DNA polymerase, and $Mg^{2+}$, and
(b) subjecting the reaction solution of (a) mentioned above to a temperature cycle to amplify DNA molecules complementary to a part or all of the nucleic acid template, wherein:
the other thermostable DNA polymerase is selected from, for example, Taq DNA polymerase, Pfu DNA polymerase, Tth DNA polymerase showing no reverse transcription activity in the presence of $Mg^{2+}$, and a combination thereof, and the nucleic acid amplification method is preferably an RT-PCR method.

The nucleic acid amplification method of the present invention may further comprise (c) monitoring progress of the nucleic acid amplification reaction in real time. "(c) Monitoring progress of the nucleic acid amplification reaction in real time" can be performed by using, but not limited to, an intercalation method (e.g., using SYBR Green I dye), hybridization method (e.g., Fret probe method, TaqMan probe method etc.), Lux primer method, and so forth. In the hybridization method, progress of the nucleic acid amplification reaction can be monitored in real time by measuring the fluorescence intensity of the reaction solution containing an oligonucleotide probe to which a fluorescent dye is attached. As the oligonucleotide probe, for example, those having a structure in which a reporter fluorescent dye is bound to the 5' end and a quencher dye to the 3' end, such as TaqMan® probes (TaqMan® probes containing Invitrogen™ TAMRA™ dye as a quencher dye, or Applied Biosystems™ TaqMan® MGB probe) can be used.

"(c) Monitoring progress of the nucleic acid amplification reaction in real time" means monitoring progress of the nucleic acid amplification reaction in real time by measuring fluorescence intensity of the reaction solution at arbitrary time points during the nucleic acid amplification reaction, and plotting the obtained fluorescence intensities against the numbers of cycles, since the fluorescence intensity increases (or decreases) depending on the amount of amplified nucleic acid. By "(c) monitoring progress of the nucleic acid amplification reaction in real time", the target nucleic acid can be quickly detected, and the target nucleic acid contained in a sample can be quantified on the basis of the amplification ratio.

According to a specific embodiment, the present invention provides a method for amplifying a nucleic acid using a reaction solution containing a DNA polymerase and $Mg^{2+}$, wherein the DNA polymerase comprises an amino acid sequence having an identity of 90% or higher to the amino acid sequence of SEQ ID NO: 5 in which amino acids at both positions 744 and 745 are replaced with positively charged amino acids. According to a specific embodiment, the present invention provides a method for amplifying a nucleic acid using a reaction solution containing a DNA polymerase and $Mg^{2+}$, wherein the DNA polymerase comprises an amino acid sequence with amino acid substitutions at amino acids at both positions 744 and 745 in the amino acid sequence of SEQ ID NO:5, the amino acid substitution at position 744 is E744K or E744R, and the amino acid substitution at position 745 is A745K or A745R. In these embodiments, the nucleic acid amplification reaction may be PCR or one-step RT-PCR.

According to a specific embodiment, the present invention provides a method for amplifying a nucleic acid using a reaction solution containing two or more kinds of DNA polymerases and $Mg^{2+}$, wherein the two or more kinds of DNA polymerases consist of at least a DNA polymerase containing an amino acid sequence having an identity of 90% or higher to the amino acid sequence of SEQ ID NO: 5, in which amino acid at position 683 is replaced with lysine, leucine or tyrosine, and amino acids at both positions 744 and 745 are replaced with positively charged amino acids, and another thermostable DNA polymerase. According to a further specific embodiment, preferably, the amino acid substitution at position 683 is E683K, the amino acid substitution at position 744 is E744K or E744R, and the amino acid substitution at position 745 is A745K or A745R. In these embodiments, the nucleic acid amplification reaction may be PCR or one-step RT-PCR.

The present invention provides a composition containing the modified DNA polymerase mentioned above. The composition of the present invention may be a composition for performing PCR or RT-PCR, preferably one-step RT-PCR. The composition of the present invention may contain a buffer, sugar, EDTA, nonionic surfactant, glycerol, and so forth, in addition to the modified DNA polymerase described above. In addition, the composition of the present invention can further contain another thermostable DNA polymerase, which can be selected from, for example, Taq DNA polymerase, Pfu DNA polymerase, Tth DNA polymerase not showing reverse transcription activity in the presence of $Mg^{2+}$, and a combination thereof.

The present invention provides a kit comprising the modified DNA polymerase mentioned above and $Mg^{2+}$. The kit of the present invention may be a kit for performing PCR or RT-PCR, preferably one-step RT-PCR. The kit of the present invention may further comprise another thermostable DNA polymerase, which may be selected from, for example, Taq DNA polymerase, Pfu DNA polymerase, Tth DNA polymerase not showing reverse transcription activity in the presence of $Mg^{2+}$, and a combination thereof. The kit of the present invention may comprise water, reagents for conducting nucleic acid reactions (e.g., dNTPs, primers, probe, monovalent ions (potassium and ammonium ions), buffer, sugar, BSA, nonionic surfactant, anti-DNA polymerase antibody (anti-Tth or anti-Taq antibody), instruction for conducting nucleic acid reactions, container, package, and so forth.

Examples

The present invention will be more specifically explained with reference to the following examples. However, the present invention is not limited by these examples.

(Reagents and equipments)

In the examples, the following reagents were used.

Vector pET15b (Merck Millipore, 69661)
*E. coli* JM109 strain (Nippon Gene, 317-06241)
Rosetta (DE3) pLysS (Merck Millipore, 70956)
Reagents for LB medium

Bacto tryptone (BD, 211705)
Bacto yeast extract (BD, 212750)
NaCl (FUJIFILM Wako Pure Chemicals, 018-01675)

Ampicillin (FUJIFILM Wako Pure Chemicals, 014-23302)
Chloramphenicol (FUJIFILM Wako Pure Chemicals, 030-19452)
IPTG (FUJIFILM Wako Pure Chemicals, 092-05324)
Imidazole (FUJIFILM Wako Pure Chemicals, 095-00015)
λDNA (Nippon Gene, 318-00414)
MgCl₂ (Nippon Gene, 310-90361)
MnCl₂ (FUJIFILM Wako Pure Chemicals, 133-00725)
dNTPs (Nippon Gene)
0.1 x ROX Passive Reference (Nippon Gene)
Anti-Taq antibody (Nippon Gene, 311-08221)

The antigen for the anti-Taq antibody is Taq DNA polymerase, thus it was selected in consideration of binding to Tth DNA polymerase in the process of screening, and it was confirmed to inhibit the polymerase activity of Tth DNA polymerase.

The primers used in the examples were synthesized by Nippon Gene Materials.

The following equipments were used in the examples.

Thermal cycler: 2720 Thermal Cycler (ThermoFisher Scientific)
Real-time PCR equipment: Applied Biosystems 7500 Fast Real-Time PCR
System (ThermoFisher Scientific), Run mode: Fast 7500

(Example 1)

Modification of expression vector

A modified pET15b2 (SEQ ID NO: 1) was created by inserting an artificially synthesized oligo DNA into a commercially available vector, pET15b, at the Nde1 site thereof.

Cloning of Tth DNA polymerase

A Tth DNA polymerase gene was amplified by PCR using a genomic DNA derived from *Thermus thermophilus* OM72 strain isolated from hot spring water in Oita Prefecture by Tokyo University of Pharmacy and Life Sciences as a template, and primers of SEQ ID NO: 2 and 3 (Table 1) designed on the basis of the sequence of *T. thermophilus* HB8, and cloned into the modified pET15b2 mentioned above at the Nde1 and Sal1 sites thereof. The cloned Tth DNA polymerase gene was sequenced, and it was confirmed that the nucleotide sequence of Tth DNA polymerase derived from *T. thermophilus* OM72 strain matched that of Tth DNA polymerase derived from *T. thermophilus* HB27 strain (SEQ ID NO: 4).

[Table 1]

| | |
|---|---|
| SEQ ID NO: 2 | gggcatATGGAGGCGATGCTTCCGC |
| SEQ ID NO: 3 | ggggtcgacCTAACCCTTGGCGGAAAGCC |

However, since the designed primer (SEQ ID NO: 3) was based on the sequence of *T. thermophilus* HB8, the nucleotide sequence for the 2502nd position of the amino acid sequence of SEQ ID NO: 4 was the same as that of the HB8 strain, not the HB27 strain. The amino acid sequence of the expressed wild-type Tth DNA polymerase (SEQ ID NO: 5) is shown in Fig. 1.

Preparation of modified Tth DNA polymerase

By using the prepared wild-type Tth DNA polymerase expression plasmid as a template, and the primers of SEQ ID NOS: 6 to 13 (Table 2), site-directed mutagenesis was performed.

[Table 2]

| Primer | Sequence (5'→3') | SEQ ID NO: |
|---|---|---|
| Tth ER-F | gaagagcgtcagggagcgcgcggagcgcatg | SEQ ID NO: 6 |
| Tth ER-R | catgcgctccgcgcgctccctgacgctcttc | SEQ ID NO: 7 |
| Tth RA-F | gaagagcgtcaggcgcgccgcgggagcgcatg | SEQ ID NO: 8 |
| Tth RA-R | catgcgctccgcggcgcgcctgacgctcttc | SEQ ID NO: 9 |
| Tth RR-F | gaagagcgtccgtcgccgcgcggagcgcatg | SEQ ID NO: 10 |
| Tth RR-R | catgcgctccgcgcggcgacggacgctcttc | SEQ ID NO: 11 |
| Tth KK-F | gaagagcgtccgtaagaaagcggagcgcatg | SEQ ID NO: 12 |
| Tth KK-R | catgcgctccgctttcttacggacgctcttc | SEQ ID NO: 13 |

The *E. coli* JM109 strain was transformed with the mutation-introduced plasmids, and mutant plasmids were prepared from the resulting colonies and sequenced to confirm that the mutations had been introduced. As a result of the mutagenesis, modified Tth DNA polymerase expression plasmids were obtained, with which the 744th and 745th amino acid residues EA of the amino acid sequence of SEQ ID NO: 5 change to ER, RA, RR, and KK, respectively.

Purification of Tth DNA polymerases

*E. coli* Rosetta (DE3) pLysS was transformed with the wild-type Tth DNA polymerase expression plasmid and the various modified Tth DNA polymerase expression plasmids. The resulting colonies were incubated at 37°C in a liquid medium obtained by supplementing ampicillin and chloramphenicol to LB until absorbance at 600 nm reached approximately 0.5, IPTG was added to a final concentration of 1 mM, and incubation was continued for further 4 hours to produce Tth DNApolymerases. The cells were suspended in 50 mM Tris-HCl (pH 8.0), 0.5 M NaCl, 1% Triton X-100, and 20 mM imidazole, and sonicated. After centrifugation, the supernatant was heat-treated at 80°C, centrifuged, and subjected to purification using HisTrap HP column (Cytiva 17524802), HiTrap Q column (Cytiva 17115401), and HiTrap Heparin column (Cytiva 17040701), and finally the medium was replaced with a storage buffer (10 mM Tris-HCl (pH 7.5), 300 mM KCl, 0.1 mM EDTA, 1 mM DTT, 0.095% Triton X-100, 50% glycerol) to obtain various Tth DNA polymerases.

(Example 2)

PCR using Tth DNA polymerase in the presence of $MgCl_2$ or $MnCl_2$

PCR was performed by using the wild-type Tth DNA polymerase (WT) or modified Tth DNA polymerase (RA, ER, RR, or KK) prepared in Example 1 to amplify a 520 bp $\lambda$DNA in the presence of $MgCl_2$ or $MnCl_2$. PCR was performed by using a thermal cycler under the following PCR conditions: 10 ng of $\lambda$DNA was added to 25 $\mu$L of a reaction solution containing 20 mM Tris-HCl (pH 8.0), 50 mM KCl, 0.1% Tween 20, 0.1 mg/mL acetylated BSA, 0.5% DMSO, 1 mM DTT, 500 mM trehalose, 2 mM $MgCl_2$ or 2 mM $MnCl_2$, 0.2 mM dNTPs, 0.4 $\mu$M primers (SEQ ID NOS: 14 and 15) for amplifying 520 bp $\lambda$DNA, one of 60 ng of the wild-type Tth DNA polymerase (WT), 60 ng of the modified Tth DNA polymerase (RA), 60 ng of the modified Tth DNA polymerase (ER), 60 ng of DNA polymerase (RR), and 60 ng of the modified Tth DNA polymerase (KK), and 0.25 $\mu$g of anti-Taq antibody, heat denaturation was performed at 95°C for 2 minutes, then a temperature cycle of 95°C for 30 seconds, $\rightarrow$ 55°C for 30 seconds $\rightarrow$ 72°C for 30 seconds was repeated 30 times, and finally extension reaction was performed at 72°C for 5 minutes.

[Table 3]

| Primers for $\lambda$DNA amplification | | |
|---|---|---|
| Primer | Sequence (5'$\rightarrow$3') | SEQ ID NO |
| Forward | GTGTTTTATCTCTGCGAGCATAATGC | SEQ ID NO: 14 |
| Reverse | CATCCACACGCAGCAGCGTCTGTTC | SEQ ID NO: 15 |

After completion of the reaction, 8.3 $\mu$L of the reaction solution was subjected to 2% agarose/TAE gel electrophoresis, staining was performed with ethidium bromide, and the presence or absence of amplification of the target DNA fragment was confirmed under UV irradiation.
The results are shown in Fig. 2. When $MgCl_2$ was added, bands were observed for all the Tth DNA polymerases. On the other hand, when $MnCl_2$ was added, no bands were confirmed for all the Tth DNA polymerases. On the basis of these results, it was confirmed that the modified Tth DNA polymerases (RA, ER, RR, and KK) prepared in Example 1 have DNA polymerase activity in the presence of $MgCl_2$, like the wild type (WT).

(Example 3)

RT-PCR using Tth DNA polymerase in the presence of $MgCl_2$ or $MnCl_2$

RT-PCR was performed by using the wild-type Tth DNA polymerase (WT) and modified Tth DNA polymerases (RA, ER, RR, and KK) prepared in Example 1 to amplify a 734 bp partial region of the mouse $\beta$-actin gene in the presence of $MgCl_2$ or $MnCl_2$. RT-PCR was performed by using a thermal cycler under the following PCR conditions: 100 ng of mouse kidney total RNA was added to 25 $\mu$L of a reaction solution containing 100 ng of total RNA extracted and purified from mouse kidney, 20 mM Tris-HCl (pH 8.8), 50 mM KCl, 0.1% Tween 20, 0.1 mg/mL acetylated BSA, 0.5 % DMSO, 1 mM DTT, 500 mM trehalose, 2 mM $MgCl_2$ or 2 mM $MnCl_2$, 0.2 mM dNTPs, 0.4 $\mu$M primers (SEQ ID NOS: 16 and 17) for amplifying a 734 bp partial region of the mouse $\beta$-actin gene, one of 60 ng of the wild-type Tth DNA polymerase (WT), 60 ng of the modified Tth DNA polymerase (RA), 60 ng of the modified Tth DNA polymerase (ER), 60 ng of the modified Tth DNA polymerase (RR), and 60 ng of the modified Tth DNA polymerase (KK), and 0.25 μg of anti-Taq antibody, reverse transcription reaction was performed at 65°C for 30 minutes, then thermal denaturation was performed at 95°C for 2 minutes, a cycle of 95°C for 30 seconds → 60°C for 30 seconds → 72°C for 30 seconds was repeated 35 times, and finally extension reaction was performed at 72°C for 5 minutes.

[Table 4]

| Primers for amplifying partial region of mouse β-actin gene | | |
|---|---|---|
| Primer | Sequence (5'→3') | SEQ ID NO |
| Forward | ATGACGATATCGCTGCGCTGGTC | SEQ ID NO: 16 |
| Reverse | CTGGCCGTCAGGCAGCTCATAG | SEQ ID NO: 17 |

After the reaction was completed, 8.3 μL of the reaction solution was subjected to 2% agarose/TAE gel electrophoresis, staining was performed with ethidium bromide, and the presence or absence of amplification of the target DNA fragment was confirmed under UV irradiation.

Fig. 3 shows the results of RT-PCR in which the 734 bp region was amplified by using the wild-type Tth DNA polymerase (WT) and modified Tth DNA polymerases (RA, ER, RR, and KK) in the presence of $MgCl_2$ or $MnCl_2$. When $MgCl_2$ was added, bands were confirmed only for the modified Tth DNA polymerases in which mutations were introduced for two amino acids (RR and KK). In contrast, when $MnCl_2$ was added, no bands were observed for all the Tth DNA polymerases. It is considered that this was because the reverse transcription activity of the wild-type Tth DNA polymerase is exhibited in the presence of $Mn^{2+}$, but the polymerase activity thereof is exhibited only in the presence of $Mg^{2+}$, and therefore DNA was not amplified from cDNA to such a level that bands thereof become visible.

From these results, it was confirmed that the modified Tth DNA polymerases (RR and KK) having the amino acid sequence of the wild-type Tth DNA polymerase in which both the 744th and 745th amino acid residues were replaced have reverse transcription activity in the presence of $MgCl_2$, which the wild-type Tth DNA polymerase (WT, the amino acid residues are EA) does not have. It is also inferred that the reverse transcription activity exhibited in the presence of $MgCl_2$ is a function obtained by replacing both the 744th and 745th amino acid residues, because no bands were confirmed with the modified Tth DNA polymerases in which one of the 744th and 745th amino acid residues was replaced (RA and ER).

In Examples 2 and 3, the PCR and RT-PCR reactions were performed with the addition of the anti-Taq antibody. The anti-Taq antibody was added in the hope of improving specificity and sensitivity. The inventors of the present invention have confirmed that the same results as in Examples 2 and 3 were obtained without the addition of anti-Taq antibody.

A 295 bp partial region of the β-actin gene was amplified by one-step RT-PCR (performed in the presence of $MgCl_2$) using 0.1 to 100 ng of HeLa cell total RNA as a template. RT-PCR was performed by using a thermal cycler under the following PCR conditions: 0.1, 1, 10, or 100 ng of the HeLa cell total RNA was added to 25 μL of a reaction solution containing 20 mM Tris-HCl (pH 8.8), 50 mM KCl, 0.1% Tween 20, 0.1 mg/mL acetylated BSA, 0.5% DMSO, 1 mM DTT, 500 mM trehalose, 2 mM $MgCl_2$, 0.2 mM dNTPs, 0.4 μM primers for amplifying a 295 bp partial region of the human β-actin gene (5'-TCACCCACACTGTGCCCATCTACGA-3 ' (SEQ ID NO: 23) and 5'-CAGCGGAACCGCTCATTGCCAATGG-3' (SEQ ID NO: 24)), one of 60 ng of the wild-type Tth DNA polymerase (WT), 65 ng of the modified Tth DNA polymerase (RR), and 60 ng of the modified Tth DNA polymerase (KK) (WT and RR, and WT and KK were mixed at a ratio of 1:1), and 0.5 μg of anti-Taq antibody, the reverse transcription reaction was performed at 65°C for 10 minutes, then thermal denaturation was performed at 95°C for 2 minutes, and further a cycle of 95°C for 30 seconds → 60°C for 60 seconds was repeated 40 times. As a result, it was demonstrated that the addition of the antibody to the reaction solution enables the amplification even with a smaller amount of the template compared with the case where the antibody is not added, as shown in Fig. 3B. The addition of WT that shows strong activity during PCR slightly increases the sensitivity in the absence of the antibody. On the other hand, when the antibody was added, the effect of the addition of WT is not clearly observed.

(Example 4)

Real-time PCR using Tth DNA polymerase

By using the wild-type Tth DNA polymerase and modified Tth DNA polymerases prepared in Example 1, real-time PCR was performed in a detection system for detecting SARS-CoV-2 (National Institute of Infectious Diseases, Pathogen Detection Manual 2019 - nCoV Ver. 2.9.1, N set No. 2 (N2 set)). The real-time PCR was performed by using Applied Biosystems 7500 Fast Real-Time PCR System in the run mode of Fast 7500 under the following PCR conditions: a positive control DNA (for N2 set) obtained by subjecting the plasmid DNA (SEQ ID NO: 21) containing TE (pH 8.0) and a target sequence of N set No. 2 to SspI and EcoO109I restriction enzyme treatment was added in an amount of 50, 500, 5,000, or 50,000 copies to 20 $\mu$L of a reaction solution containing 10 mM Tris-HCl (pH 9.0), 100 mM KCl, 0.025% Triton X-100, 250 mM trehalose, 2 mM $MgCl_2$, 0.2 mM dNTPs, 0.1 x ROX Passive Reference (Nippon Gene), 0.5 $\mu$M primer NIID_2019-nCOV_N_F2 (SEQ ID NO: 18), 0.7 $\mu$M primer NIID_2019-nCOV_N_R2 (SEQ ID NO: 19), 0.2 $\mu$M TaqMan probe obtained by labeling the 5' end and 3' end of NIID_2019-nCOV_N_P2 (SEQ ID NO: 20) with FAM and TAMRA, respectively, one of 46 ng of the wild-type Tth DNA polymerase (WT), 15 ng of the modified Tth DNA polymerase (KK), and 25.6 ng of the modified Tth DNA polymerase (RR), and 0.1 $\mu$g of anti-Taq antibody, thermal denaturation was performed at 95°C for 20 seconds, and a cycle of 95°C for 3 seconds → 60°C for 30 seconds was repeated 45 times. It is expected that the addition of the anti-Taq antibody can provide an effect of improving specificity and sensitivity (Japanese Patent Unexamined Publication (Kokai) No. 2008-17787).

[Table 5]

| Primer | Sequence (5'→3') | SEQ ID NO |
|---|---|---|
| NIID_2019-nCOV_N_F2 | AAATTTTGGGGACCAGGAAC | SEQ ID NO: 18 |
| NIID_2019-nCOV_N_R2 | TGGCAGCTGTGTAGGTCAAC | SEQ ID NO: 19 |
| NIID_2019-nCOV_N_P2 | ATGTCGCGCATTGGCATGGA | SEQ ID NO: 20 |

After the reaction was completed, analysis was performed with a threshold line: 0.5 and baseline: 3 to 15 cycles, and the Ct value was calculated. The Tth DNA polymerases were added in such amounts that the reaction systems to which the Tth DNApolymerases were added should provide the same Ct values (WT, 46 ng; KK, 15 ng; and RR, 25.6 ng), which were determined by preliminarily performing real-time PCR to amplify 500 copies of the control DNA.

Fig. 4 shows the calibration curves for the amplification curves of the real-time PCR performed in the presence of $MgCl_2$ and anti-Taq antibody.

Table 6 summarizes the results including the Ct values and PCR efficiencies.

[Table 6]

| | Copy number of positive control DNA | WT(EA) | KK | RR |
|---|---|---|---|---|
| Ct value | 50000 | 23.07 | 23.09 | 23.06 |
| | 5000 | 26.25 | 26.46 | 26.50 |
| | 500 | 29.77 | 29.87 | 29.98 |
| | 50 | 33.19 | 33.28 | 33.06 |
| | TE (pH 8.0) | - | - | - |
| Calibration curve | Slope | -3.387 | -3.398 | -3.349 |
| | PCR efficiency | 97.3% | 96.9% | 98.9% |
| | R2 Value | 0.997 | 1.000 | 0.998 |

PCR amplification efficiencies were calculated from calibration curves prepared by using serially diluted DNA templates. The appropriate range of the PCR amplification efficiency is considered to be 80 to 120%, and it was confirmed that both the wild-type Tth DNA polymerase (WT) and the modified Tth DNA polymerases (KK and RR) show PCR amplification efficiencies falling within the appropriate range. In PCR performed by using DNA as the template, no significant difference was observed in Ct value or PCR efficiency between the wild-type Tth DNA polymerase (WT) and the modified Tth DNA polymerases (KK and RR).

(Example 5)

One-step RT-qPCR using Tth DNA polymerase in the presence of $MgCl_2$ or $MnCl_2$

By using the wild-type Tth DNA polymerase and modified Tth DNA polymerases prepared in Example 1, one-step real-time PCR was performed in a detection system for detecting SARS-CoV-2 (National Institute of Infectious Diseases, Pathogen Detection Manual 2019-nCoV Ver. 2.9.1, N set No. 2 (N2 set)). The one-step RT-qPCR was performed by using Applied Biosystems 7500 Fast Real-Time PCR System in the run mode of Fast 7500 under the following PCR conditions: a positive control RNA containing DW and a target sequence of N set No. 2 (SEQ ID NO: 22) was added in an amount of 50, 500, or 5,000 copies to 20 $\mu$L of a reaction solution containing 10 mM Tris-HCl (pH 9.0), 100 mM KCl, 0.025% Triton X-100, 250 mM trehalose, 2 mM $MgCl_2$ or 2 mM $MnCl_2$, 0.2 mM dNTPs, 0.1 x ROX Passive Reference (Nippon Gene), 0.5 $\mu$M primer NIID_2019-nCOV_N_F2 (SEQ ID NO: 18), 0.7 $\mu$M primer NIID_2019-nCOV_N_R2 (SEQ ID NO: 19), 0.2 $\mu$M TaqMan probe obtained by labeling the 5' end and 3' end of NIID_2019-nCOV_N_P2 (SEQ ID NO: 20) with FAM and TAMRA, respectively, and one of 46 ng of the wild-type Tth DNA polymerase (WT), 15 ng of the modified Tth DNA polymerase (KK), and 25.6 ng of the modified Tth DNA polymerase (RR), reverse transcription reaction was allowed at 65°C for 10 minutes, then thermal denaturation was performed at 95°C for 20 seconds, and further a cycle of 95°C for 3 seconds → 60°C for 30 seconds was repeated 45 times. After completion of the reaction, analysis was performed with a threshold line: 0.5 and baseline: 3 to 15 cycles, and the Ct value was calculated.

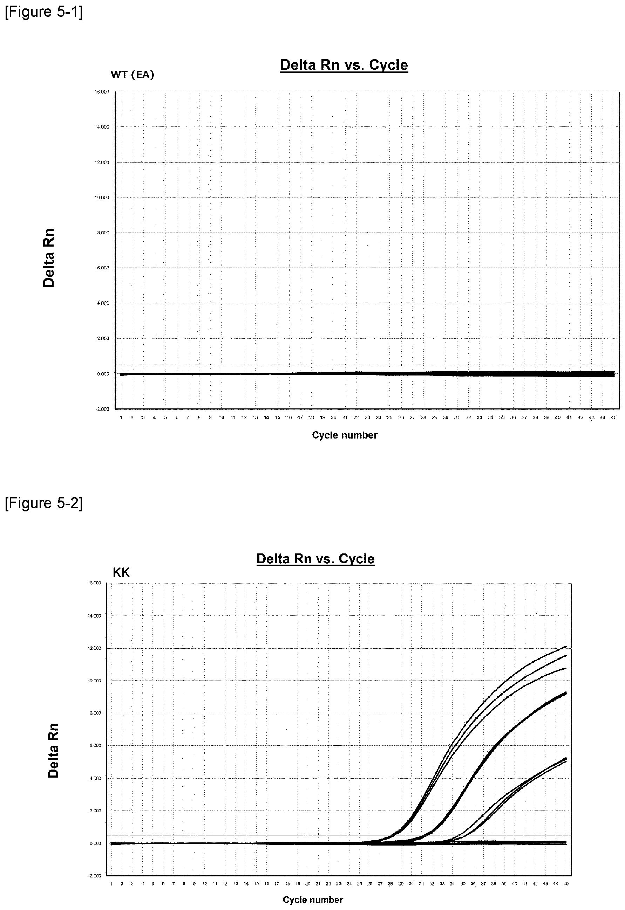

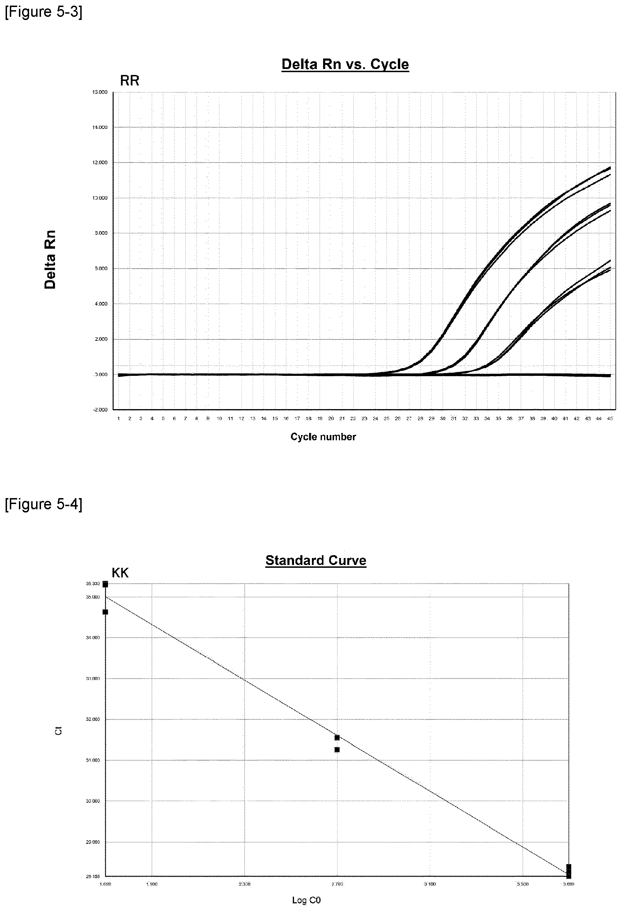

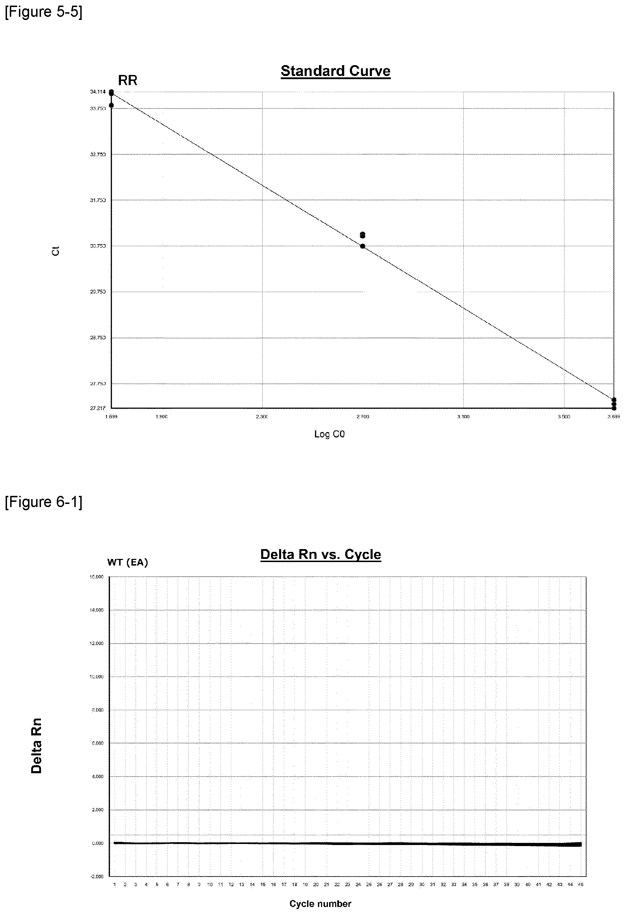

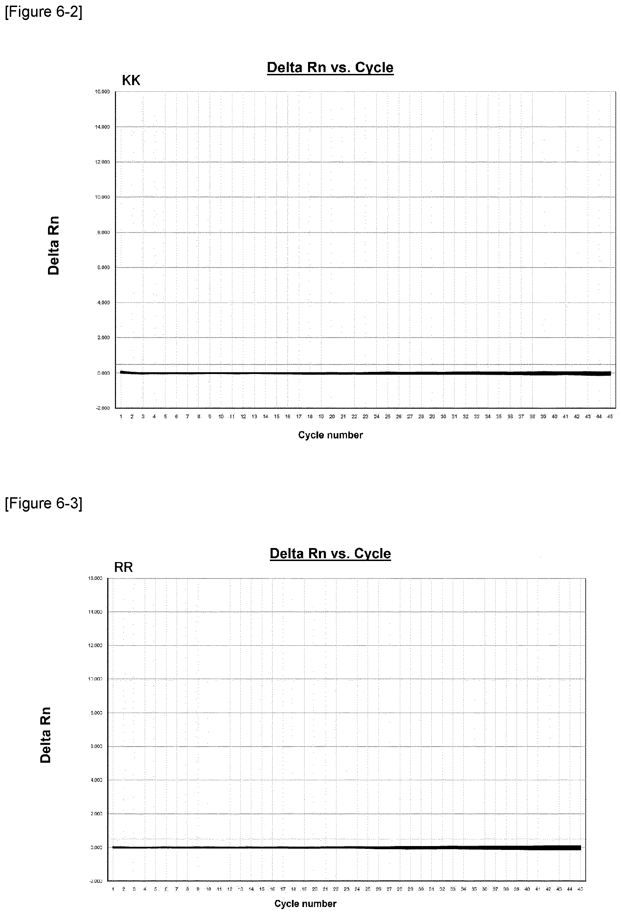

In the one-step RT-qPCR performed in the presence of $MnCl_2$, no rise was observed for the amplification curves for the wild-type Tth DNA polymerase (WT) and the modified Tth DNA polymerases (KK and RR) (Fig. 6). In the one-step RT-qPCR performed in the presence of $MgCl_2$, no rise was observed for the amplification curve of the wild-type Tth DNA polymerase (WT), while up to 50 copies were detected for the modified Tth DNA polymerases (KK and RR) (Fig. 5).

The results of the Ct values and PCR efficiencies observed in the one-step RT-qPCR performed (in the presence of $MgCl_2$) in the SARS-CoV-2 detection system are summarized in Table 7. From these results, it was confirmed that the modified Tth DNA polymerases (RR and KK) have reverse transcription activity in the presence of $MgCl_2$, which the wild-type Tth DNA polymerase (WT) does not have, and can be used for one-step RT-qPCR in a reaction solution containing $MgCl_2$.

[Table 7]

| | Copy number. of positive control DNA | WT(EA) | KK | RR |
|---|---|---|---|---|
| Ct value | 5000 | - | 28.28 | 27.31 |
| | 500 | - | 31.45 | 30.91 |
| | 50 | - | 35.08 | 34.00 |
| | DW | - | - | - |
| Calibration curve | Slope | - | -3.403 | -3.348 |
| | PCR efficiency | - | 96.7% | 98.9% |
| | R2 Value | - | 0.993 | 0.997 |

PCR amplification efficiencies were calculated from calibration curves prepared by using serially diluted DNA templates. The appropriate range of the PCR amplification efficiency is considered to be 80 to 120%, and it was confirmed that both the wild-type Tth DNA polymerase (WT) and the modified Tth DNA polymerases (KK and RR) show PCR amplification efficiencies falling within the appropriate range.

(Example 6)

One-step RT-qPCR using Tth DNA polymerase in the presence of $MgCl_2$ or $MnCl_2$ under conditions of adding anti-Taq antibody

In this example, one-step RT-qPCR was performed by using the same template, primers and Tth DNA polymerases as in Example 5, but anti-Taq antibody was added to the reaction solution, unlike Example 5.

By using the wild-type Tth DNA polymerase and modified Tth DNA polymerases prepared in Example 1, one-step RT-qPCR was performed in the presence of $MgCl_2$ or $MnCl_2$ in a detection system for detecting SARS-CoV-2 (National Institute of Infectious Diseases, Pathogen Detection Manual 2019-nCoV Ver. 2.9.1, N set No. 2 (N2 set)). The one-step RT-qPCR was performed by using Applied Biosystems 7500 Fast Real-Time PCR System in the run mode of Fast 7500 under the following PCR conditions: a positive control RNA containing DW and a target sequence of N set No. 2 (SEQ ID NO: 22) was added in an amount of 50, 500, 5,000, or 50,000 copies to 20 $\mu$L of a reaction solution containing 10 mM Tris-HCl (pH 9.0), 100 mM KCl, 0.025% Triton X-100, 250 mM trehalose, 2 mM $MgCl_2$ or 2 mM $MnCl_2$, 0.2 mM dNTPs, 0.1 x ROX Passive Reference (Nippon Gene), 0.5 $\mu$M primer NIID_2019-nCOV_N_F2 (SEQ ID NO: 18), 0.7 $\mu$M primer NIID_2019-nCOV_N_R2 (SEQ ID NO: 19), 0.2 $\mu$M TaqMan probe obtained by labeling the 5' end and 3' end of NIID_2019-nCOV_N_P2 (SEQ ID NO: 20) with FAM and TAMRA, respectively, one of 46 ng of the wild-type Tth DNA polymerase (WT), 15 ng of the modified Tth DNA polymerase (KK), and 25.6 ng of the modified Tth DNA polymerase (RR), and 0.1 μg of anti-Taq antibody, reverse transcription reaction was allowed at 65°C for 30 minutes, then thermal denaturation was performed at 95°C for 2 seconds, and further a cycle of 95°C for 3 seconds → 60°C for 30 seconds was repeated 45 times. After completion of the reaction, analysis was performed with a threshold line: 0.5 and baseline: 3 to 15 cycles, and the Ct value was calculated.

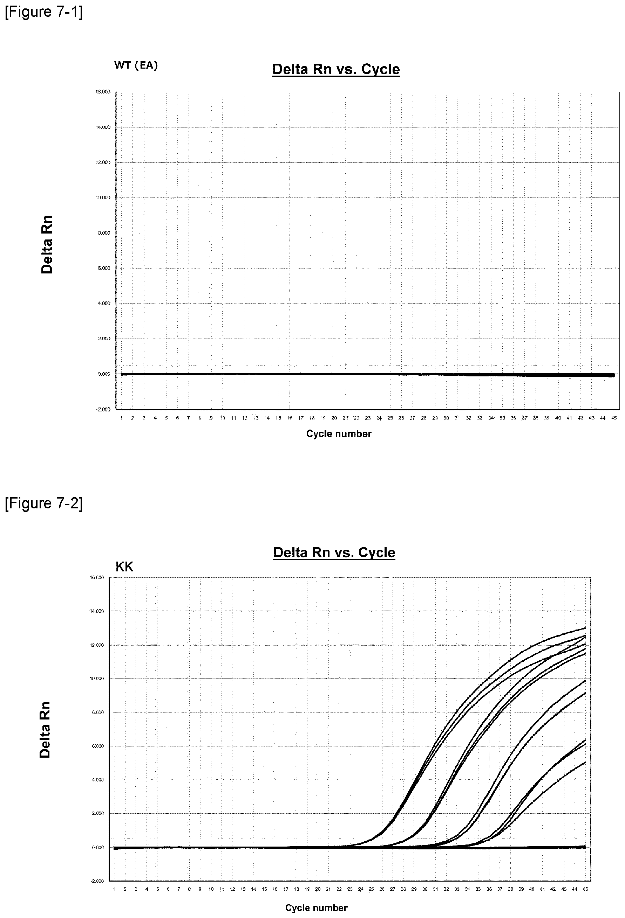

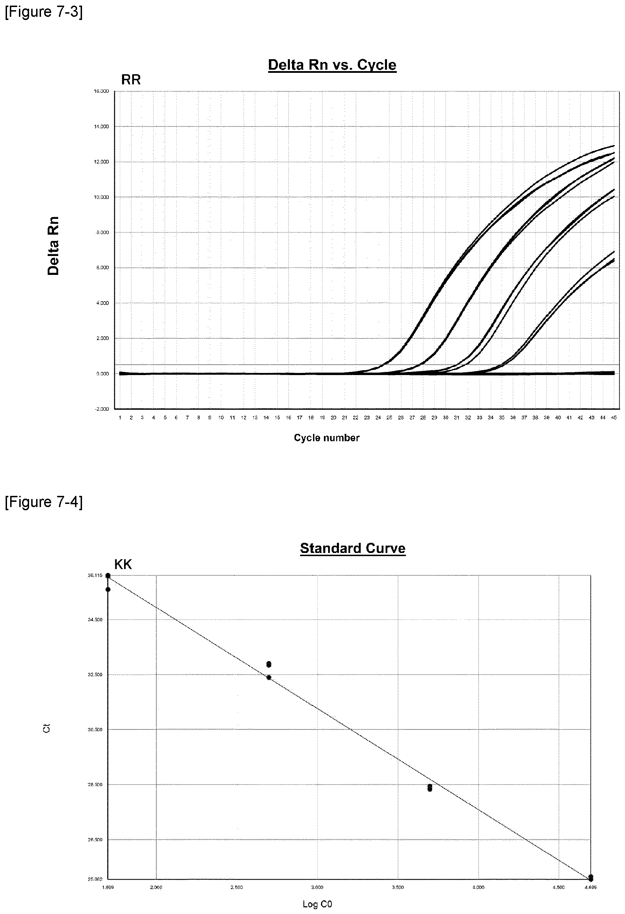

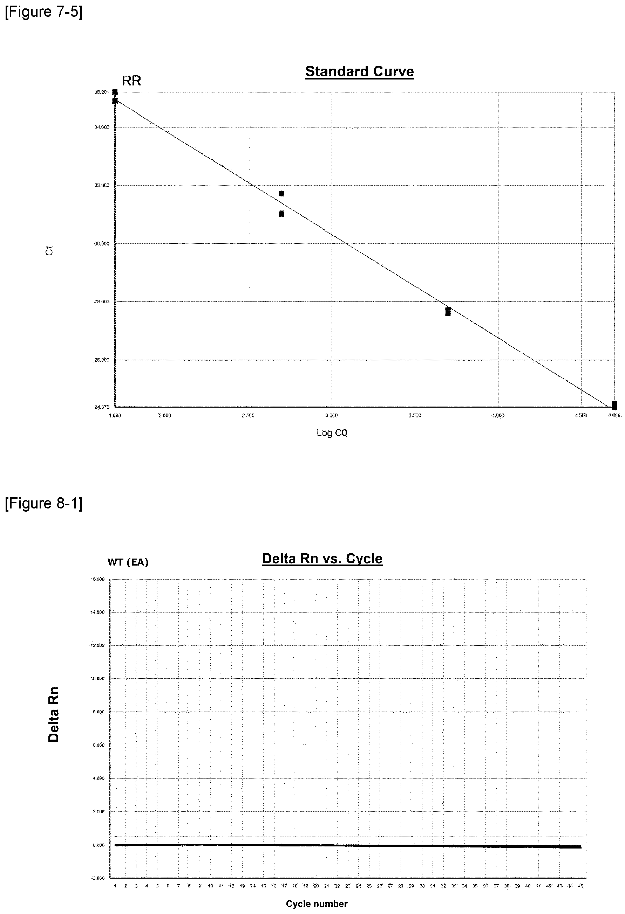

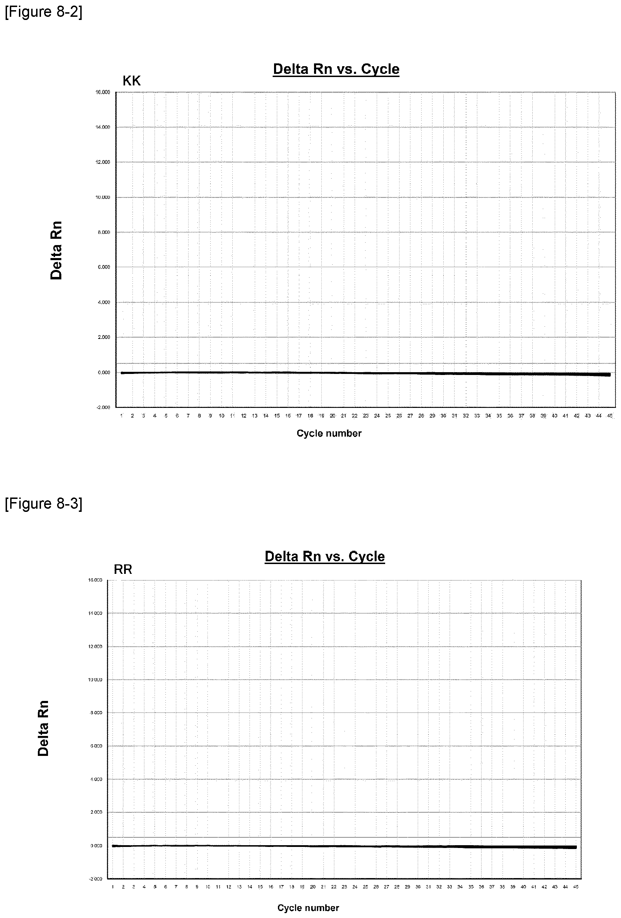

In the one-step RT-qPCR performed in the presence of $MnCl_2$, no rise was observed for amplification curves for both the wild-type Tth DNA polymerase (WT) and the modified Tth DNA polymerases (KK and RR) (Fig. 8). In the one-step RT-qPCR performed in the presence of $MgCl_2$, no rise was observed for the amplification curves for the wild-type Tth DNA polymerase (WT), while up to 50 copies were detected for the modified Tth DNA polymerases (KK and RR) (Fig. 7).

The results of the Ct values and PCR efficiencies observed in one-step RT-qPCR performed (in the presence of $MgCl_2$) in the SARS-CoV-2 detection system are summarized in Table 8. From these results, it was confirmed that the modified Tth DNA polymerases (RR and KK) have reverse transcription activity in the presence of $MgCl_2$, which the wild-type Tth DNA polymerase (WT) does not have, and can be used for one-step RT-qPCR in a reaction solution containing $MgCl_2$.

[Table 8]

| | Copy number of positive control RNA | WT(EA) | KK | RR |
|---|---|---|---|---|
| Ct value | 50000 | - | 25.12 | 24.42 |
| | 5000 | - | 28.38 | 27.66 |
| | 500 | - | 32.72 | 31.24 |
| | 50 | - | 35.93 | 35.09 |
| | DW | - | - | - |
| Calibration curve | Slope | - | -3.676 | -3.561 |
| | PCR efficiency | - | 87.1% | 90.9% |
| | R2 Value | - | 0.995 | 0.996 |

PCR amplification efficiencies were calculated from calibration curves prepared by using serially diluted RNA templates. The appropriate range of the PCR amplification efficiency is considered to be 80 to 120%, and it was confirmed that both the wild-type Tth DNA polymerase (WT) and the modified Tth DNA polymerases (KK and RR) provide PCR amplification efficiencies falling within the appropriate range.

Since results equivalent to those of Example 5 were obtained even under the condition that the anti-Taq antibody was added, it was confirmed that the reverse transcription activity of the modified Tth DNA polymerases (RR and KK) observed in the presence of $MgCl_2$ was not affected by the presence or absence of the anti-Taq antibody.

(Example 7)

One-step RT-qPCR using modified Tth DNA polymerase having further amino acid substitution at position 683

Modified Tth DNA polymerases having a further amino acid substitution at position 683 in addition to the substitution of the amino acid residues KK at positions 744 and 745 for EA in the amino acid sequence of SEQ ID NO: 5 were prepared and purified. KKK, YKK, and LKK expression vectors were prepared by using a kit sold by TOYOBO (KOD-Plus-Mutagenesis Kit). The expression vectors were prepared according to the instructions of the manufacturer of the kit, provided that the number of cycles was changed to 5, and primers listed in Table 9 were used. Purification was performed as described in Example 1.

[Table 9]

| TthE683K-F | AAActtgccatcccctacgaggagg | SEQ ID NO: 25 |
|---|---|---|
| TthE683L-F | CT Gcttgccatcccctacgaggagg | SEQ ID NO: 26 |
| TthE683Y-F | TATcttgccatcccctacgaggagg | SEQ ID NO: 27 |

(continued)

| TthE683-R | ctgggagagcctatgggcggac | SEQ ID NO: 28 |
|---|---|---|

Hereafter, the modified Tth DNA polymerases having an amino acid substitution of K, L, and Y at position 683 are referred to as KKK, LKK, and YKK, respectively.

By using the wild-type Tth DNA polymerase and modified Tth DNA polymerases (KK and KKK), one-step RT-qPCR was performed in a detection system for detecting SARS-CoV-2 in the presence of $MgCl_2$ (National Institute of Infectious Diseases, Pathogen Detection Manual 2019-nCoV Ver. 2.9.1, N set No. 2 (N2 set)). The instrument used was ProFlex™ PCR System (Thermo Fisher scientific).

The composition (final concentrations) of the reaction solution was as follows: 10 mM Tris-HCl (pH 9.0), 100 mM KCl, 0025% Triton X-100, 250 mM trehalose, 0.2 mM dNTPs, 2 mM $MgCl_2$, 0.1 x ROX Reference Dye, 1 ng/ml of Tth DNA polymerase (KK, 1 ng/ml modified Tth (KK); KK + WT, 0.5 ng/ml modified Tth (KK) + 0.5 ng/ml wild-type Tth; and KKK + WT, 0.5 ng/ml modified Tth (KKK) + 0.5 ng/ml wild-type Tth). As the template, purified RNA extracted from a sample of inactivated coronavirus diluted 1000-fold with saliva by using ISOSPIN Viral RNA was used.

The primers shown in Table 10 were used.

[Table 10]

| Primer | Sequence (5'→3') | SEQ ID NO |
|---|---|---|
| 2019-nCoV_N2-F | TTACAAACATTGGCCGCAAA | SEQ ID NO: 29 |
| 2019-nCoV_N2-R | GCGCGACATTCCGAAGAA | SEQ ID NO: 30 |

The composition of the PCR reaction solution and PCR cycle were as follows. PCR reaction solution

```
PCR reaction solution
    2x Mix          10.0 μl    PCR cycle     (Ramp Rate : 3.5 °C /sec)
    10 μM F-Primer   1.0 μl    65°C 10 min
    10 μM R-Primer   1.0 μl    95°C 20 sec
    Template         4.0 μl    95°C 3 sec  ⎫  ×45
    DW               4.0 μl    60°C 30 see ⎭  Cycles
    _____
    Total           20.0 μl
```

After completion of the reaction, the reaction solution was subjected to 3% agarose S/TAE gel electrophoresis, staining was performed with ethidium bromide, and the presence or absence of amplification of the target DNA fragment was confirmed under UV irradiation.

The results are shown in Fig. 9. When the wild-type Tth DNA polymerase (WT) and the modified Tth DNA polymerase (KKK) were used in combination (WT + KKK), a band was confirmed in gel electrophoresis at the size of amplified template of 67 bp. In the case of samples containing a lot of contaminating nucleic acids (e.g., RNA extracted from saliva-derived samples etc.), a decrease in specificity was observed in some cases, but with WT + KKK, no nonspecific amplification bands appeared in the gel electrophoresis, and the target template was amplified. It is considered that this was because the modified Tth DNA polymerase (KKK) maintains the reverse transcription activity in the presence of $MgCl_2$, but the reduced DNA polymerase activity thereof suppresses the amplification of nonspecific nucleic acids.

Further, a 734 bp portion of β-actin was amplified from total RNA extracted from mouse kidney by using modified KKK, LKK and YKK Tth DNA polymerases. The primers and cycle used were the same as in Example 3. The RT-PCR reaction was performed in a volume of 20 μL. The buffer composition (final concentration) was as follows: 10 mM Tris-HCl (pH 9.0), 100 mM KCl, 0.05% Tween 20, 0.05 mg/mL BSA, 1.5 mM $MgCl_2$, 0.5 M trehalose, and 0.2 mM for each of dNTPs. The amount of enzyme used was 40 ng.

The results of RT-PCR in which the 734 bp portion of β-actin was amplified are shown in Fig. 10. As a result, it was confirmed that the PCR activities of all the modified DNA polymerases were weakened. It was suggested that the E683 mutation in the KK type modified enzyme resulted in an enzyme characterized in that it shows weakened PCR activity while it maintains the reverse transcription activity.

Free Text of Sequence Listing

SEQ ID NO: 1: Modified pET15b2
SEQ ID NOS: 2 and 3: Primers used for cloning of Tth DNA polymerase
SEQ ID NO: 4: Nucleotide sequence of Tth DNA polymerase
SEQ ID NO: 5: Amino acid sequence of Tth DNA polymerase
SEQ ID NOS: 6 to 13: Primers used for the preparation of modified Tth DNA polymerases
SEQ ID NOS: 14 and 15: Primers for amplification of λDNA
SEQ ID NOS: 16 and 17: Primers for amplification of a partial region of mouse β-actin gene
SEQ ID NOS: 18 to 20: Primers for detection system for detecting SARS-CoV-2
SEQ ID NO: 21: Plasmid DNA containing the target sequence of N set No. 2
SEQ ID NO: 22: Positive control RNA containing the target sequence of N set No. 2 SEQ ID NOS: 23 and 24: Primers for amplification of a partial region of human β-actin gene
SEQ ID NOS: 25 to 28: Primers used for the preparation of modified Tth DNA polymerases
SEQ ID NOS: 29 and 30: Primers for a detection system for detecting SARS-CoV-2

**Claims**

1. A DNA polymerase having an activity to catalyze a reverse transcription reaction in the presence of $Mg^{2+}$ and containing an amino acid sequence having an identity of 90% or higher to the amino acid sequence of SEQ ID NO: 5 and amino acid substitutions at both positions of 744 and 745 with positively charged amino acids.

2. The DNA polymerase according to claim 1, wherein:

    the amino acid substitution at position 744 is E744K, E744R or E744H, and
    the amino acid substitution at position 745 is A745K, A745R or A745H.

3. A DNA polymerase having an activity to catalyze a reverse transcription reaction in the presence of $Mg^{2+}$, and comprising an amino acid sequence with amino acid substitutions at both positions of 744 and 745 in the amino acid sequence of SEQ ID NO: 5, wherein:

    the amino acid substitution at position 744 is E744K or E744R, and
    the amino acid substitution at position 745 is A745K or A745R.

4. The DNA polymerase according to any one of claims 1 to 3 having a further amino acid substitution at position 683 in the amino acid sequence the amino acid sequence of SEQ ID NO: 5.

5. The DNA polymerase according to claim 4, wherein the amino acid substitution at position 683 is E683K, E683L or E683Y.

6. A method for amplifying a nucleic acid, which comprises:

    (a) preparing a reaction solution containing a nucleic acid template, the DNA polymerase according to any one of claims 1 to 5, and $Mg^{2+}$, and
    (b) subjecting the reaction solution of (a) to a temperature cycle to amplify a DNA molecule complementary to a part or all of the nucleic acid template.

7. The method according to claim 6, wherein in the step of (a), the reaction solution further contains another thermostable DNA polymerase.

8. The method according to claim 6 or 7, which further comprises (c) monitoring progress of the nucleic acid amplification reaction in real time.

9. The method according to any one of claims 6 to 8, wherein the nucleic acid amplification reaction is a one-step RT-PCR.

10. A composition containing the DNA polymerase according to any one of claims 1 to 5.

11. A kit comprising the DNA polymerase according to any one of claims 1 to 5 and Mg2+.

12. A nucleic acid encoding the DNA polymerase according to any one of claims 1 to 5.

13. An expression vector or host cell containing the nucleic acid according to claim 12.

[Figure 1]

Amino acid sequence of wild-type Tth DNA polymerase (SEQ ID NO:5)

```
MEAMLPLFESKGRVLLVDGHHLAYRTFFALKGLTTSRGEPVQAVYGFAKS
LLKALKEDGYKSVFVVFDAKAPSFRHEAYEAYKAGRAPTPEDFPRQLALI
KELVDLLGFTRLEVPGYEADDVLATLAKKAEKEGYEVRILTADRDLYQLV
SDRVAVLHPEGHLITPEWLWEKYGLRPEQWVDFRALVGDPSDNLPGVKGI
GEKTALKLLKEWGSLESLLKNLDRVKPENVREKIKAHLEDLRLSLELSRV
RADLPLEVDLAQGREPDREGLRAFLERLEFGSLLHEFGLLEAPTPLEEAP
WPPPEGAFVGFVLSRPEPMWAELKALAACRDGRVHRAEDPLAGLGDLEEV
RGLLAKDLAVLALREGLDLAPGDDPMLLAYLLDPSNTTPEGVARRYGGEW
TEDAAHRALLSERLHRNLLKRLEGEEKLLWLYHEVEKPLSRVLAHMEATG
VRLDVAYLQALSLELAEEIRRLEEEVFRLAGHPFNLNSRDQLERVLFDEL
RLPALGKTQKTGKRSTSAAVLEALREAHPIVEKILQHRELTKLKNTYVDP
LPSLVHPRTGRLHTRFNQTATATGRLSSSDPNLQNIPVRTPLGQRIRRAF
VAEAGWALVALDYSQIELRVLAHLSGDENLIRVFQEGKDIHTQTASWMFG
VPPEAVDPLMRRAAKTVNFGVLYGMSAHRLSQELAIPYEEAVAFIERYFQ
SFPKVRAWIEKTLEEGRKRGYVETLFGRRRYVPDLNARVKSVREAAERMA
FNMPVQGTAADLMKLAMVKLFPRLREMGARMLLQVHDELLLEAPQARAEE
VAALAKEAMEKAYPLAVPLEVEVGIGEDWLSAKG*
```

[Figure 2]

[Figure 3A]

[Figure 3B]

[Figure 4-1]

WT (EA)　　　　　**Delta Rn vs. Cycle**

Cycle number

[Figure 4-2]

**Delta Rn vs. Cycle**

KK

Cycle number

[Figure 4-3]

**Delta Rn vs. Cycle**

RR

Cycle number

[Figure 4-4]

WT (EA)          **Standard Curve**

Log C0

[Figure 4-5]

[Figure 4-6]

[Figure 5-1]

WT (EA)

**Delta Rn vs. Cycle**

Delta Rn

Cycle number

[Figure 5-2]

KK

**Delta Rn vs. Cycle**

Delta Rn

Cycle number

[Figure 5-3]

Delta Rn vs. Cycle

RR

Delta Rn

Cycle number

[Figure 5-4]

Standard Curve

KK

Ct

Log C0

[Figure 5-5]

**Standard Curve**

RR

Ct

Log C0

[Figure 6-1]

**Delta Rn vs. Cycle**

WT (EA)

Delta Rn

Cycle number

[Figure 6-2]

**Delta Rn vs. Cycle**

KK

[Figure 6-3]

**Delta Rn vs. Cycle**

RR

[Figure 7-1]

WT (EA)

## Delta Rn vs. Cycle

[Figure 7-2]

KK

## Delta Rn vs. Cycle

[Figure 7-3]

**Delta Rn vs. Cycle**

RR

[Figure 7-4]

**Standard Curve**

KK

[Figure 7-5]

[Figure 8-1]

[Figure 8-2]

**Delta Rn vs. Cycle**

KK

[Figure 8-3]

**Delta Rn vs. Cycle**

RR

[Figure 9]

[Figure 10]

## INTERNATIONAL SEARCH REPORT

| International application No. |
| --- |
| **PCT/JP2021/048076** |

| A. | CLASSIFICATION OF SUBJECT MATTER |
| --- | --- |

*C12N 15/54*(2006.01)i; *C12M 1/00*(2006.01)i; *C12N 1/15*(2006.01)i; *C12N 1/19*(2006.01)i; *C12N 1/21*(2006.01)i; *C12N 5/10*(2006.01)i; *C12N 9/12*(2006.01)i; *C12N 15/63*(2006.01)i; *C12Q 1/6844*(2018.01)i
FI:  C12N15/54 ZNA; C12M1/00 A; C12N1/15; C12N1/19; C12N1/21; C12N5/10; C12N9/12; C12N15/63 Z; C12Q1/6844 Z

According to International Patent Classification (IPC) or to both national classification and IPC

| B. | FIELDS SEARCHED |
| --- | --- |

Minimum documentation searched (classification system followed by classification symbols)

C12N15/54; C12M1/00; C12N1/15; C12N1/19; C12N1/21; C12N5/10; C12N9/12; C12N15/63; C12Q1/6844

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN); UniProt/GeneSeq

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2015-156858 A (AGILENT TECHNOLOGIES, INC.) 03 September 2015 (2015-09-03) paragraph [0012], etc. | 1-13 |
| A | JP 2009-136188 A (PRECISION SYSTEM SCIENCE CO LTD) 25 June 2009 (2009-06-25) table 1, etc., example 5 | 1-13 |
| A | WO 2017/090684 A1 (UNIV KYUSHU NAT UNIV CORP) 01 June 2017 (2017-06-01) entire text | 1-13 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **18 January 2022** | **15 February 2022** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

**INTERNATIONAL SEARCH REPORT**

International application No.

**PCT/JP2021/048076**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT
### Information on patent family members

International application No.

**PCT/JP2021/048076**

| Patent document cited in search report | | | Publication date (day/month/year) | Patent family member(s) | | | Publication date (day/month/year) |
|---|---|---|---|---|---|---|---|
| JP | 2015-156858 | A | 03 September 2015 | US | 2015/0232821 | A1 | |
| | | | | paragraph [0010], etc. | | | |
| JP | 2009-136188 | A | 25 June 2009 | (Family: none) | | | |
| WO | 2017/090684 | A1 | 01 June 2017 | US | 2018/0346889 | A1 | |
| | | | | entire text | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020216600 A
- WO 1994008032 A

- JP 5191041 B
- JP 2008017787 A

**Non-patent literature cited in the description**

- **T.W MYERS ; D.H. GELFAND.** *Biochem.,* 1991, vol. 30, 7661-7666
- **ARAKAWA et al.** *DNA Research,* 1996, vol. 3, 87-92

- **SUN et al.** *Proteins,* 01 October 2006, vol. 65 (1), 231-8
- **WANG et al.** *Nucleic Acids Research,* 2004, vol. 32 (3), 1197-1207